(19) Europäisches Patentamt European Patent Office Office européen des brevets

(11) **EP 4 276 467 B1**

(12) **EUROPEAN PATENT SPECIFICATION**

(45) Date of publication and mention of the grant of the patent:
**29.01.2025 Bulletin 2025/05**

(21) Application number: **23172990.6**

(22) Date of filing: **12.05.2023**

(51) International Patent Classification (IPC):
*G01N 33/58* (2006.01)    *G01N 33/86* (2006.01)

(52) Cooperative Patent Classification (CPC):
**G01N 33/86; G01N 33/582;** G01N 2333/755

(54) **METHOD FOR OBTAINING INFORMATION ON VON WILLEBRAND FACTOR, MEASUREMENT SAMPLE PREPARATION METHOD, AND USE OF REAGENT KIT**

VERFAHREN ZUR GEWINNUNG VON INFORMATIONEN ÜBER DEN VON-WILLEBRAND-FAKTOR, VERFAHREN ZUR HERSTELLUNG EINER MESSPROBE UND VERWENDUNG EINES REAGENZIENKITS

PROCÉDÉ D'OBTENTION D'INFORMATIONS SUR LE FACTEUR DE VON WILLEBRAND, PROCÉDÉ DE PRÉPARATION D'ÉCHANTILLON DE MESURE ET UTILISATION D'UN KIT DE RÉACTIFS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **13.05.2022 JP 2022079618**

(43) Date of publication of application:
**15.11.2023 Bulletin 2023/46**

(73) Proprietors:
• **National University Corporation Hokkaido University Sapporo-shi, Hokkaido 060-0808 (JP)**
• **SYSMEX CORPORATION Kobe-shi Hyogo 651-0073 (JP)**

(72) Inventors:
• **KINJO, Masataka Sapporo-shi, Hokkaido, 060-0808 (JP)**
• **GANGULY, Akshay Kobe-shi, Hyogo, 651-0073 (JP)**
• **RAMASWAMY, Lausonia Kobe-shi, Hyogo, 651-0073 (JP)**

(74) Representative: **De Clercq & Partners Edgard Gevaertdreef 10a 9830 Sint-Martens-Latem (BE)**

(56) References cited:
**EP-A1- 3 904 881**

• **TORRES RICHARD ET AL: "Clinical Measurement of von Willebrand Factor by Fluorescence Correlation Spectroscopy", CLINICAL CHEMISTRY, vol. 58, no. 6, 1 June 2012 (2012-06-01), US, pages 1010 - 1018, XP093088162, ISSN: 0009-9147, DOI: 10.1373/ clinchem.2011.179200**
• **LIPPOK SVENJA ET AL: "Exponential Size Distribution of von Willebrand Factor", BIOPHYSICAL JOURNAL, ELSEVIER, AMSTERDAM, NL, vol. 105, no. 5, 3 September 2013 (2013-09-03), pages 1208 - 1216, XP028712252, ISSN: 0006-3495, DOI: 10.1016/ J.BPJ.2013.07.037**

**Description**

FIELD OF THE INVENTION

The present invention relates to a method for obtaining information on von Willebrand factor (VWF) size. The present invention also relates to a method for preparing a measurement sample for use in said method. The present invention also relates to use of a reagent kit in the method for obtaining information on VWF.

BACKGROUND

Von Willebrand factor, abbreviated herein as VWF, is a large-molecular-weight plasma glycoprotein and plays an important role in primary hemostasis. Specifically, VWF binds to subendothelial tissue of a damaged blood vessel at a bleeding site, where it promotes the adhesion of platelet and the formation of platelet thrombus. VWF, which in itself is a protein of about 250-kDa, multimerizes and is present in the blood as multimers of various sizes (about 500 kDa to about 15,000 kDa). A large-molecular-weight multimer of VWF has a higher activity of recruiting platelets than a low-molecular-weight multimer and is important in primary hemostasis.

Von Willebrand disease (VWD) is a congenital coagulopathy, in which primary hemostasis is impaired due to quantitative decrease, complete defect, or qualitative abnormality of VWF, and therefore bleeding tends to occur. In the diagnosis of VWD, disease types are classified by, for example, the amount, activity, and composition of multimers, of VWF. The composition of VWF multimers has been analyzed by a combination of SDS-gel electrophoresis and Western blotting using an anti-VWF antibody. This analysis allows one to determine the amount of large-, middle-, and low-molecular-weight VWF multimers, by separating them according to their molecular weights.

In recent years, attempts have been made to measure the molecular size of VWF by a fluorescence imaging technique, called fluorescence correlation spectroscopy (FCS) or fluorescence cross-correlation spectroscopy (FCCS). FCS measures the fluctuation of a fluorescence signal (for example, changes over time in the fluorescence intensity) that is caused by a molecule labeled with one kind of fluorescent substance performing Brownian motion, in or out of a minute observation region formed by a laser beam from a confocal optical system. FCCS measures the fluctuations of fluorescence signals that result from a molecule labeled with two kinds of fluorescent substances. FCS analyzes the measured fluctuation with an autocorrelation function. FCCS analyzes the simultaneity of the measured fluctuations of the two different fluorescent substances with a cross-correlation function.

FCS and FCCS allow one to obtain information, for example, on the number of molecules and molecular size. For example, Torres R. et al., Clin. Chem., vol. 58, pp. 1010-1018, 2012 describes that VWF in plasma from healthy persons and VWD patients was measured by FCS using a fluorescently-labeled anti-VWF antibody, and their VWF diffusion time measurement values were compared, where diffusion time is the size-dependent average residence time of the VWF-antibody immunocomplex in the observation region. Japanese Laid-Open Patent Publication No. 2021-173705 describes that the molecular size of recombinant VWF was measured by FCCS using two fluorescently labeled anti-VWF antibodies. Lippok et al. (2013 Biophysical Journal 150:1208-1216) describes the measurement of the size distribution of VWF by FCS using 1,5 M urea, without using polyclonal antibodies or a plurality of monoclonal antibodies or aptamers that bind to epitopes different from each other. At this urea concentration VWF can be conceived as a semi-flexible polymer as urea stretches it without affecting its basic structure.

SUMMARY OF THE INVENTION

In the measurement of an antigen by FCS and FCCS using a fluorescently labeled antibody or antibodies, respectively, their results may be affected by the concentration of the antigen in a measurement sample. This is caused by the dissociation between the fluorescently labeled antibody or antibodies and the antigen. When an antibody having a certain dissociation constant is used at a given final concentration, the number of molecules of the complex formed by the antibody and the antigen is dependent on the concentration of the antigen in the measurement sample. Therefore, the lower the concentration of the antigen in the measurement sample, the greater the influence of the dissociation between the fluorescently-labeled antibody and the antigen. Torres R. et al., Clin. Chem., vol. 58, pp. 1010-1018, 2012 also describes that the diffusion time obtained by FCS varied depending on the concentration of VWF in the measurement samples.

The diffusion time is the average time required for a fluorescently-labeled molecule to pass through a given observation region and is a parameter representing the molecular size of the molecule. A molecule with a small size passes through the observation region more quickly, and thus has a shorter diffusion time, relative to a molecule with a large size. This means that in a measurement sample, a decrease in the number of molecules of the complex due to the dissociation of the fluorescently-labeled antibody and the antigen results in a reduced value of the diffusion time for the measurement sample. Therefore, when a measurement sample has a low concentration of an antigen, it is unclear whether the information on the molecular size obtained by FCS or FCCS reflects the actual molecular size of the antigen.

It is known, on the other hand, that when a fluorescent substance itself or beads each having a fluorescent substance covalently bonded thereto (fluorescent beads) are measured by FCS, the measurement results of the molecular size obtained from measurement samples hardly vary even though the measurement samples have different concentrations of the fluorescent substance or the fluorescent beads (See, e.g., Gendron P-O. et al.,J. Fluoresc., vol. 18, pp. 1093-1101, 2008, and Yamamoto J. et al., Opt. Exp., vol. 27, pp. 14835-14841, 2019). The present inventors also have confirmed that when VWF having two fluorescent substances covalently bonded thereto was measured by FCCS, the diffusion times for measurement samples were almost constant even though these measurement samples had different concentration of VWF.

However, there is a need for indirect labeling of VWF with a fluorescently-labeled capturing agent or agents, in order to measure VWF in a biological sample collected from a subject, by FCS or FCCS, respectively. The concentration of VWF in a biological sample collected from a subject can be different from that from a different subject. Thus, the present inventors aim to provide a means for reducing the influence of the concentration of VWF in the measurement of VWF by FCS and FCCS using a fluorescently-labeled capturing agent or agents, respectively.

The present invention provides a method for obtaining information on VWF size, comprising the following steps: denaturing, with urea, VWF contained in a biological sample; fluorescently labeling the denatured VWF using a capturing agent that comprises a fluorescent substance and binds to the denatured VWF; and obtaining information on the size of the fluorescently-labeled VWF by FCS or FCCS; wherein when the information is obtained by FCS, the capturing agent comprises a polyclonal antibody, or a plurality of monoclonal antibodies or aptamers that bind to epitopes different from each other; and wherein when the information is obtained by FCCS, the capturing agent comprises a first capturing agent that comprises a first fluorescent substance and binds to the denatured VWF, and a second capturing agent that comprises a second fluorescent substance and binds to the denatured VWF, wherein the second fluorescent substance is one having a maximum fluorescence emission in a wavelength range different from that of the first fluorescent substance, and wherein the first capturing agent that comprises the first fluorescent substance and binds to the denatured VWF is a polyclonal antibody that comprises the first fluorescent substance, or a plurality of monoclonal antibodies or aptamers that each comprise the first fluorescent substance and bind to epitopes different from each other.

The present invention further provides a method for preparing a measurement sample for use in FCS or FCCS, comprising the following steps: denaturing, with urea, VWF contained in a biological sample; and fluorescently labeling the denatured VWF using a capturing agent that comprises a fluorescent substance and binds to the denatured VWF; wherein when the VWF fluorescently labeled with the capturing agent is used for measurement by FCS, the capturing agent comprises a polyclonal antibody, or a plurality of monoclonal antibodies or aptamers that bind to epitopes different from each other; and wherein when the VWF fluorescently labeled with the capturing agent is used for measurement by FCCS, the capturing agent comprises a first capturing agent that comprises a first fluorescent substance and binds to the denatured VWF, and a second capturing agent that comprises a second fluorescent substance and binds to the denatured VWF, wherein the second fluorescent substance is one having a maximum fluorescence emission in a wavelength range different from that of the first fluorescent substance, and wherein the first capturing agent that comprises the first fluorescent substance and binds to the denatured VWF is a polyclonal antibody that comprises the first fluorescent substance, or a plurality of monoclonal antibodies or aptamers that each comprise the first fluorescent substance and bind to epitopes different from each other.

The present invention further provides a reagent kit for use in the above-described methods in which FCS is used, said kit comprising urea and a capturing agent that comprises a fluorescent substance and binds to urea-denatured VWF, wherein the capturing agent comprises a polyclonal antibody or a plurality of monoclonal antibodies or aptamers that bind to epitopes different from each other.

The present invention also provides use of a reagent kit in the above-described methods in which FCCS is used, said kit comprising urea, a first capturing agent that comprises a first fluorescent substance and binds to urea-denatured VWF, and a second capturing agent that comprises a second fluorescent substance and binds to the urea-denatured VWF, wherein the second fluorescent substance is one having a maximum absorption in a wavelength region different from that of the first fluorescent substance, and the first capturing agent that comprises the first fluorescent substance and binds to the urea-denatured VWF is a polyclonal antibody that comprises the first fluorescent substance, or a plurality of monoclonal antibodies or aptamers that each comprise the first fluorescent substance and bind to epitopes different from each other.

According to the present invention, the influence of the concentration of VWF can be reduced in the measurement of VWF by FCS and FCCS using a fluorescently-labeled capturing agent or agents, respectively.

BRIEF DESCRIPTION OF THE DRAWINGS

Fig. 1A is a schematic representation showing an example of a reagent kit according to an embodiment of the present invention that can be used for the measurement of VWF by FCS.

Fig. 1B is a schematic representation showing an example of a reagent kit according to an embodiment of the present invention that can be used for the measurement of VWF by FCCS.

Fig. 2A is a graph showing diffusion times by FCCS from measurement samples comprising different concentrations of VWF having Alexa Fluor® 488 and Alexa Fluor® 647 each covalently bonded thereto.

Fig. 2B is a graph showing diffusion times by FCCS from measurement samples comprising different concentrations of VWF having an Alexa Fluor® 488-labeled monoclonal antibody and an Alexa Fluor® 647-labeled monoclonal antibody each bonded thereto.

Fig. 3A is a graph showing diffusion times by FCS from measurement samples comprising different concentrations of non-denatured or urea-denatured VWF, which was indirectly labeled with an Alexa Fluor® 488-labeled monoclonal antibody.

Fig. 3B is a graph showing diffusion times by FCS from measurement samples comprising different concentrations of non-denatured or urea-denatured VWF, which was indirectly labeled with an Alexa Fluor® 647-labeled monoclonal antibody.

Fig. 4 is a graph showing diffusion times by FCS from measurement samples comprising different concentrations of urea-denatured VWF, which was indirectly labeled with an Alexa Fluor® 488-labeled polyclonal antibody.

Fig. 5 is a graph showing diffusion times by FCS from measurement samples comprising different concentrations of urea-denatured VWF, which was indirectly labeled with two Alexa Fluor® 488-labeled monoclonal antibodies.

Fig. 6 is a graph showing diffusion times by FCCS from measurement samples comprising different concentrations of non-denatured or urea-denatured VWF, which was indirectly labeled with an Alexa Fluor® 488-labeled monoclonal antibody and an Alexa Fluor® 647-labeled monoclonal antibody.

Fig. 7 is a graph showing diffusion times by FCCS from measurement samples comprising different concentrations of urea-denatured VWF, which was indirectly labeled with an Alexa Fluor® 488-labeled polyclonal antibody and an Alexa Fluor® 647-labeled monoclonal antibody.

Fig. 8 is a graph showing diffusion times by FCCS from measurement samples comprising different concentrations of urea-denatured VWF, which was indirectly labeled with two Alexa Fluor® 488-labeled monoclonal antibodies and an Alexa Fluor® 647-labeled monoclonal antibody.

Fig. 9 is a graph showing diffusion times by FCCS from measurement samples comprising different concentrations of VWF denatured with urea at given concentrations, which was indirectly labeled with an Alexa Fluor® 488-labeled polyclonal antibody and an Alexa Fluor® 647-labeled polyclonal antibody.

Fig. 10 is a graph showing diffusion times by FCCS from measurement samples comprising different concentrations of urea-denatured VWF, which was indirectly labeled with reagents comprising given concentration ratios of an Alexa Fluor® 488-labeled polyclonal antibody and an Alexa Fluor® 647-labeled polyclonal antibody.

Fig. 11 represents the results of an analysis of non-agitated or agitated standard human plasma by SDS-gel electrophoresis and Western blotting, and an example of a densitometric analysis of the blot.

Fig. 12A is a graph showing diffusion times by FCS from measurement samples comprising urea-denatured VWF with different ratios of a large-molecular-weight fraction (LMW indices), which was indirectly labeled with an Alexa Fluor® 488-labeled polyclonal antibody.

Fig. 12B is a graph showing diffusion times by FCS from measurement samples comprising urea-denatured VWF with different LMW indices, which was indirectly labeled with an Alexa Fluor® 647-labeled polyclonal antibody.

Fig. 12C is a graph showing diffusion times by FCCS from measurement samples comprising urea-denatured VWF with different LMW indices, which was indirectly labeled with an Alexa Fluor® 488-labeled polyclonal antibody and an Alexa Fluor® 647-labeled polyclonal antibody.

Fig. 13A is a graph showing diffusion times by FCS from measurement samples prepared from non-agitated or agitated standard human plasma (SHP) and comprising different concentrations of urea-denatured VWF, which was indirectly labeled with an Alexa Fluor® 488-labeled monoclonal antibody.

Fig. 13B is a graph showing diffusion times by FCS from measurement samples prepared from non-agitated or agitated standard human plasma and comprising different concentrations of urea-denatured VWF, which was indirectly labeled with an Alexa Fluor® 647-labeled monoclonal antibody.

Fig. 13C is a graph showing diffusion times by FCCS from measurement samples prepared from non-agitated or agitated standard human plasma and comprising different concentrations of urea-denatured VWF, which was indirectly labeled with an Alexa Fluor® 488-labeled monoclonal antibody and an Alexa Fluor® 647-labeled monoclonal antibody.

Fig. 14 is a graph showing diffusion times by FCS from measurement samples prepared from non-agitated or agitated standard human plasma and comprising different concentrations of urea-denatured VWF, which was indirectly labeled with an Alexa Fluor® 488-labeled polyclonal antibody.

Fig. 15 is a graph showing diffusion times by FCCS from measurement samples prepared from non-agitated or agitated standard human plasma and comprising different concentrations of urea-denatured VWF, which was indirectly labeled with an Alexa Fluor® 488-labeled polyclonal antibody and an Alexa Fluor® 647-labeled monoclonal antibody.

## DETAILED DESCRIPTION OF THE PREFERRED EMBODIMENTS

In a method for obtaining information on VWF size according to a first aspect of the present invention (which is also referred to as hereinafter an "information obtaining method according to the present invention"), VWF contained in a biological sample is first denatured with urea. In this specification, the terms "von Willebrand factor" and "VWF" include a monomer and multimers of VWF of any molecular size. A VWF multimer is formed from two or more VWF monomers. The VWF multimer can be of any form, as long as it comprises two or more VWF monomers, and may comprise another concrete component, such as a platelet. In the VWF multimer, the VWF monomers may not be bound strongly to each other, for example, by covalent bonding. The VWF multimer includes aggregates in which two or more VWF monomers are assembled together by looser binding. Preferably, the VWF is one that is expressed in a living body of a human being and contained in a biological sample collected from the living body.

The biological sample may be a specimen collected from a subject and comprising VWF, or a sample prepared from the specimen. The biological sample is, for example, whole blood, plasma, and serum. When whole blood or plasma is used, an anticoagulant may be added thereto. The anticoagulant is not particularly limited in its type, and is, for example, sodium citrate, an ethylenediaminetetraacetic acid (EDTA) potassium salt, an EDTA sodium salt, and a heparin salt. Among these, sodium citrate is preferable. If necessary, the biological sample may be diluted with a suitable aqueous solvent. Such aqueous solvents are, for example, water, physiological saline, and a buffer. The buffer is, for example, phosphate-buffered saline (PBS), Tris-HCl, and a Good's buffer.

The urea denaturing of VWF contained in a biological sample can be carried out, for example, by mixing urea and the biological sample. The urea which is added to the biological sample may be in solution or in solid. It is preferable to use a urea solution in terms of convenience of handling. The solvent of the urea solution is not particularly limited as long as it can dissolve urea and preferably is an aqueous solvent described above. The concentration of the urea solution is not particularly limited, and it would be sufficient if it is, for example, not less than 1 M and not more than 8 M. "M" is the unit of molar concentration and stands for "mol/L" or "mol/dm$^3$." The urea denaturing of VWF contained in a biological sample is carried out for the purpose of increasing the number of capturing agents that are to bind per molecule of VWF. It is considered that urea acts on VWF, leading to an alteration in the higher-order structure of the VWF as a protein so that in denatured VWF, more epitopes to which the capturing agent is to bind are exposed than in non-denatured VWF.

The denaturing of VWF with urea is preferably carried out in the presence of urea at a concentration of not less than 0.5 M and not more than 1.75 M. In the denaturing, the lower limit of the concentration of urea in a mixture of a biological sample and urea can be, for example, not less than 0.5 M, 0.6 M, 0.7 M, 0.8 M, or 0.9 M. The upper limit of the concentration of urea in a mixture of a biological sample and urea can be, for example, not more than 1.75 M, 1.7 M, 1.6 M, 1.5 M, 1.4 M, 1.3 M, 1.2 M, or 1.1 M.

The denaturing can be carried out, for example, by mixing a biological sample and urea and then incubating the resulting mixture at a temperature of not lower than 20°C and not higher than 45°C, preferably not lower than 30°C and not higher than 40°C. The incubation time can be, for example, not less than 5 minutes and not more than 240 minutes, and preferably not less than 10 minutes and not more than 120 minutes.

The denatured VWF is then fluorescently labeled using a capturing agent that comprises a fluorescent substance and binds to the denatured VWF (which is also referred to hereinafter as a "fluorescently-labeled capturing agent"). The fluorescently-labeled capturing agent can be obtained by binding of a capturing agent that binds to denatured VWF, and a fluorescent substance. The type of the capturing agent is a polyclonal antibody, a plurality of monoclonal antibodies that bind to epitopes different from each other, a plurality of aptamers that bind to epitopes different from each other, and a combination thereof. It is considered that since the fluorescently-labeled capturing agent comprises a polyclonal antibody, a plurality of monoclonal antibodies that bind to epitopes different from each other, a plurality of aptamers that bind to epitopes different from each other, and a combination thereof, the capturing agent can bind to more than one epitope on the molecule of VWF. This can result in an increased number of capturing agents that are to bind per molecule of VWF. The plurality of monoclonal antibodies that bind to epitopes different from each other comprise at least a first monoclonal antibody that binds to a first epitope of VWF and a second monoclonal antibody that binds to a second epitope of VWF. The plurality of aptamers that bind to epitopes different from each other comprise at least a first aptamer that binds to a first epitope of VWF and a second aptamer that binds to a second epitope of VWF.

In this specification, the term "antibody" includes a full-length antibody and a fragment thereof. The antibody fragment is, for example, Fab, Fab', F(ab')2, Fd, Fd', Fv, a light chain, a variable region of a heavy chain antibody derived from a camelid animal (VHH), a variable region of a heavy chain antibody derived from cartilaginous fish (VNAR), reduced IgG (rIgG), and a single chain antibody (scFv). The antibody is not particularly limited in its source, and can be one derived from any animal, including a mammal such as a mouse, a rat, a hamster, a rabbit, a goat, a horse, a camel, or cartilaginous fish like a shark. The isotype of the antibody may be any of IgG, IgM, IgE, IgA, or others, and preferably is IgG. The antibody that binds to the denatured VWF may be a commercially available anti-VWF antibody, or an antibody that is produced by a method known in the art. The aptamer may be a peptide aptamer or a nucleic acid aptamer. The aptamer can be produced by a known method, such as a SELEX method.

The fluorescent substance is, for example, a fluorescent dye, such as fluorescein isothiocyanate (FITC), rhodamine, Cy2®, Cy3®, Cy5®, and a series of Alexa Fluor®, and a fluorescent protein, such as a green fluorescent protein (GFP, EGFP, etc.), a yellow fluorescent protein (YFP, EYFP, etc.), a blue fluorescent protein (BFP, CFP, ECFP, etc.) and a red fluorescent protein (dsRed, mCherry, etc.), and the like. Among these, a fluorescent dye is preferable.

When information on the size of fluorescently labeled VWF is obtained by FCS, a capturing agent is used that is labeled with one kind of fluorescent substance. The one kind of fluorescent substance may be a single fluorescent substance or a combination of two or more fluorescent substances that each emit fluorescence at approximately the same wavelength. Such a combination is, for example, a combination of FITC (having a fluorescence wavelength of 522 nm) and Alexa Fluor® 488 (having a fluorescence wavelength of 519 nm), a combination of rhodamine (having a fluorescence wavelength of 570 nm) and Cy3® (having a fluorescence wavelength of 570 nm), a combination of Cy5® (having fluorescence wavelength of 667 nm) and Alexa Fluor® 647 (having a fluorescence wavelength of 665 nm), and the like.

When the above-described information is obtained by FCCS, two capturing agents can be used that are labeled with each of two kinds of fluorescent substances. That is, the capturing agent used in FCCS comprises a first capturing agent that comprises a first fluorescent substance and binds to the denatured VWF (which is also referred to hereinafter as a "first fluorescently-labeled capturing agent") and a second capturing agent that comprises a second fluorescent substance and binds to the denatured VWF (which is also referred to hereinafter as a "second fluorescently-labeled capturing agent"). Since FCCS detects two different fluorescence signals at the same time, the second fluorescent substance is one having a maximum fluorescence emission in a wavelength range different from that of the first fluorescent substance. The maximum fluorescence emission is an emission that is emitted by the excited fluorescent substance at a wavelength giving the highest fluorescence intensity, which is also referred to as a maximum fluorescence wavelength. For example, the maximum fluorescence emission of the second fluorescent substance may be in a wavelength range in which it is 30 nm or more, preferably 40 nm or more, away from that of the first fluorescent substance. The maximum fluorescence emission of a fluorescent substance can be easily examined by analyzing the fluorescence spectrum of the fluorescent substance with a known spectrofluorometer. More preferably, the second fluorescent substance is one that emits fluorescence different in color from the first fluorescent substance. There is no particular limitation on the color of the fluorescence emitted by each of the fluorescent substances: for example, the first fluorescent substance can be one that emits green fluorescence (at a wavelength from about 500 nm to about 550 nm), and the second fluorescent substance can be one that emits yellow fluorescence (at a wavelength from about 580 nm to about 600 nm) or red fluorescence (at a wavelength from about 610 nm to about 780 nm). In particular embodiments, the first fluorescently-labeled capturing agent comprises a different capturing agent than the second fluorescently-labeled capturing agent. In particular embodiments, there is at least one epitope of the denatured VWF which is recognized by only one of the first and second fluorescently labeled capturing agents.

When the above-described information is obtained by FCCS, particularly wherein at least one of FITC, Cy2®, and Alexa Fluor® 488 is used as the first fluorescent substance, it is possible to use, as the second fluorescent substance, at least one of rhodamine, Cy3®, Cy5®, and Alexa Fluor® 647. Each of the first and second fluorescent substances may be a single fluorescent substance. Alternatively, each of the first and second fluorescent substances may be a combination of two or more fluorescent substances that each emit fluorescence at approximately the same wavelength.

When the above-described information is obtained by FCCS, it is preferable that at least one of the first fluorescently-labeled capturing agent and the second fluorescently-labeled capturing agent is a polyclonal antibody, a plurality of monoclonal antibodies that bind to epitopes different from each other, a plurality of aptamers that bind to epitopes different from each other, or a combination thereof. For example, the first fluorescently-labeled capturing agent can be a polyclonal antibody that comprises the first fluorescent substance, or a plurality of monoclonal antibodies or aptamers that each comprise the first fluorescent substance and bind to epitopes different from each other, and/or the second fluorescently-labeled capturing agent can be a polyclonal antibody that comprises the second fluorescent substance, or a plurality of monoclonal antibodies or aptamers that each comprise the second fluorescent substance and bind to epitopes different from each other. In cases when any one of the first fluorescently-labeled capturing agent and the second fluorescently-labeled capturing agent is a polyclonal antibody, or a plurality of monoclonal antibodies or aptamers that bind to epitopes different from each other, the other may be a monoclonal antibody.

Labeling a capturing body with a fluorescent substance can be carried out, for example, by covalent binding of the fluorescent substance and the capturing agent by a known method, such as by an amine coupling method or by a maleimide method. The capturing agent may be fluorescently labeled using a commercially available labeling kit, a crosslinker, or the like. A fluorescent substance having a reactive group suitable for labeling is also commercially available. For example, a fluorescent substance having an N-hydroxysuccinimide (NHS) ester, a sulfodichlorophenol (SDP) ester, or a tetrafluorophenyl (TFP) ester as a reactive group can be used to label a capturing agent by the amine coupling reaction between the ester and an amino group of the capturing agent. A fluorescent substance having a maleimide group as a reactive group can be used to label a capturing agent by the reaction between the maleimide group and a sulfhydryl group of the capturing agent. When the capturing agent is a monoclonal antibody and the fluorescent substance is a fluorescent protein, the capturing agent that comprises the fluorescent substance can be a fusion protein of the monoclonal antibody and the fluorescent protein. Such a fusion protein can be produced by a genetic recombination technique known in the art.

When the above-described information is obtained by FCS, the fluorescent labeling of the denatured VWF with a fluorescently labeled capturing agent can be carried out by mixing a mixture of a biological sample and urea, and the fluorescently labeled capturing agent. The fluorescently-labeled capturing agent is preferably in solution. When the above-described information is obtained by FCCS, the fluorescent labeling of the denatured VWF with two fluorescently labeled capturing agents can be carried out by preparing a mixture of a urea-containing biological sample, a first fluorescently-labeled capturing agent, and a second fluorescently-labeled capturing agent. The mixture obtained by this fluorescent labeling step, which comprises the biological sample, urea, and the fluorescently-labeled capturing agent or agents, is hereinafter also referred to as a "measurement sample."

The fluorescent labeling of the denatured VWF is preferably carried out in the presence of urea at a concentration of not less than 0.2 M and not more than 1 M. When the fluorescently-labeled capturing agent is used as a solution, the amount at which it is added to a mixture of a biological sample and urea is preferably one such that the concentration of urea in the measurement sample is not less than 0.2 M and not more than 1 M. In the measurement sample, the denaturing action of urea also extends to the fluorescently-labeled capturing agent or agents, but it is considered that there is substantially no influence on the measurement by FCS and by FCCS if the concentration of urea in the measurement sample is within the above-described range.

There is no particular limitation on the concentration of the fluorescently-labeled capturing agent or agents in the measurement sample. For example, when the fluorescently-labeled capturing agent is a fluorescently-labeled antibody, the fluorescently-labeled antibody can be added to a mixture of a biological sample and urea such that the final concentration of the antibody in the measurement sample is not less than 1 nM and not more than 100 nM, preferably not less than 5 nM and not more than 75 nM, and more preferably not less than 10 nM and not more than 50 nM. When the first fluorescently-labeled capturing agent and the second fluorescently-labeled capturing agent are fluorescently-labeled antibodies, it would be sufficient if the fluorescently-labeled antibodies are added such that the final concentration of each of these antibodies in the measurement sample is within the above-described range.

The fluorescent labeling can be carried out, for example, by mixing a mixture of a biological sample and urea, and a capturing agent or agents and then incubating the resulting measurement sample at a temperature of not lower than 20°C and not higher than 40°C, preferably not lower than 25°C and not higher than 37°C. The incubation time can be, for example, not less than 1 minutes and not more than 120 minutes, and preferably not less than 3 minutes and not more than 60 minutes.

In another embodiment, the denaturing of VWF with urea and the fluorescent labeling of the urea-denatured VWF with a fluorescently-labeled capturing agent or agents may be carried out substantially at the same time. In this case, a biological sample, urea, and a fluorescently-labeled antibody or antibodies are mixed to prepare a measurement sample. These may be mixed substantially at the same time. Alternatively, a biological sample and urea may be first mixed, followed by addition of a fluorescently-labeled antibody or antibodies, to prepare a measurement sample. The concentration of urea in the measurement sample is preferably not less than 0.5 M and not more than 1 M. The measurement sample comprising urea at such a concentration makes it possible that VWF is denatured with urea and the urea-denatured VWF is fluorescently labeled with a fluorescently-labeled antibody or antibodies. The denaturing and the fluorescent labeling can be carried out, for example, by mixing a biological sample and urea, and a capturing agent or agents, and then incubating the resulting measurement sample at a temperature of not lower than 20°C and not higher than 45°C, preferably not lower than 30°C and not higher than 40°C. The incubation time can be, for example, not less than 10 minutes and not more than 120 minutes, and preferably not less than 15 minutes and not more than 60 minutes.

After the denatured VWF is fluorescently labeled, information on the size of the fluorescently-labeled VWF is obtained by FCS or FCCS. The information on the size of the fluorescently-labeled VWF is not particularly limited, and include the diffusion time, the hydrodynamic radius, the hydrodynamic diameter, the volume, and the like, of the fluorescently-labeled VWF. The hydrodynamic radius, the hydrodynamic diameter, and the volume of the fluorescently-labeled VWF can be calculated from its diffusion time. The hydrodynamic radius and the hydrodynamic diameter are usually expressed in units of nanometers (nm). The volume is usually expressed in units of cubic nanometers ($nm^3$). Among these, the diffusion time of the fluorescently-labeled VWF is preferably obtained. As described above, the diffusion time of a fluorescently-labeled molecule obtained by FCS or FCCS varies according to its size, and thus is information reflecting its molecular size.

The diffusion time is the average time required for the fluorescently-labeled VWF in a measurement sample to pass through an observation region formed by a laser beam from a confocal optical system. The diffusion time can also be translated into the residence time during which the fluorescently-labeled VWF diffuses into and remains in the observation region. The diffusion time is generally expressed by the symbol "$\tau_D$" and in units of microseconds ($\mu s$) or milliseconds (ms).

Measurement and obtaining of diffusion times by FCS and FCCS are known per se: FCS and FCCS are explained in various references, for example, Eigen M. and Rigler R., Proc. Natl. Acad. Sci. USA. vol. 91, pp. 5740-5747, 1994; Bacia K. et al., Nat. Methods, vol. 3, pp. 83-89, 2006; Bacia K. and Schwille P., Nat. Protoc., vol. 2, pp. 2842-2856, 2007.

The measurement of a fluorescent molecule by FCS or FCCS can be carried out by an apparatus capable of forming an observation region by a laser beam from the confocal optical system thereof and measuring a fluorescence signal generated from the fluorescent molecule entering or exiting the observation region. Such an apparatus is known and, for example, is a confocal laser microscope, a fluorescence correlation spectroscopic analyzer, and the like. Specifically, a Carl Zeiss LSM 710 confocal laser microscope (Zeiss), an FCS Compact (Hamamatsu Photonics K.K.), and an FCS-101 (TOYOBO CO., LTD.) are commercially available, for example. These commercially available apparatuses are provided with an analysis software such as Zen software (Zeiss), allowing for analyzing the detected fluorescence signal with an autocorrelation function and with a cross-correlation function.

Measurement by FCS or FCCS using a confocal laser microscope detects a fluorescence signal or signals, respectively, for example, by dispensing a measurement sample into a 384-well glass-bottom plate and irradiating it with a laser beam. The temperature of the measurement sample during the measurement is preferably in the order of 23°C to 25°C, for example. The wavelength of the laser beam is not particularly limited as long as the fluorescent substance or substances in the measurement sample can be excited. In the measurement, the laser beam emitted from the light source of the apparatus is focused to form a measurement region having, for example, a volume in the order of 0.1 to 1 fL (femtoliter). The apparatus detects a fluorescence signal or signals which is/are resulted from the fluorescently-labeled VWF when it passes through the measurement region. In FCS, a fluorescent substance is excited using one laser beam, and a fluorescence signal generated from the excited fluorescent substance is continuously obtained. In FCCS, a first and a second fluorescent substances are excited using two laser beams having wavelengths different from each other, and a fluorescence signal generated from each of the first and second fluorescent substances that have been excited is continuously obtained. The measurement time can be set as appropriate by one skilled in the art and, for example, can be set to 10 seconds or more. The measurement may be made more than one per measurement sample. Raw data of the detected fluorescence signal is a group of data expressed in fluorescence intensity (Hz) and measurement time (s). The raw data may be processed to eliminate a measurement value representing a baseline drift or a burst (a fluorescence signal having three times or more brightness than an average fluorescence brightness).

The measurement by FCS obtains raw data from the measurement of photons of a single wavelength. The raw data is subjected to autocorrelation analysis, thereby to obtain an autocorrelation function curve. Fitting is done with a fitting model appropriate to the autocorrelation function curve, thereby to obtain a correlation curve given by a correlation function $G(\tau)$ and correlation time $\tau$ (also referred to as lag time). In FCS, fitting using an autocorrelation function can be done in a two-component model. A first component used in the two-component model can be, for example, an unreacted fluorescently-labeled capturing agent. A second component can be a complex between the denatured VWF and a fluorescently-labeled capturing agent. For example, on an analysis software such as Zen software, a one-component model is selected, in which the diffusion time of the first component is measured for the fluorescently-labeled capturing agent. On the same software, a two-component model is then selected, in which the diffusion time of the first component is fixed to that of the first component that has been measured in the one-component model and the diffusion time of the second component is measured.

The measurement by FCCS obtains raw data from the measurement of photons of each of the two wavelengths. The raw data is subjected to cross-correlation analysis, thereby to obtain a cross-correlation function curve. For each of the two wavelengths, the raw data may also be subjected to autocorrelation analysis, thereby to obtain an autocorrelation function curve for each of the first and the second fluorescent materials. Fitting is done with a fitting model appropriate to the cross-correlation function curve, thereby to obtain a correlation curve given by a correlation function $G(\tau)$ and correlation time $\tau$. In FCCS, fitting using a cross-correlation function can be done in a one-component model. The one component can be a complex between the denatured VWF and two fluorescently-labeled capturing agents. For example, on an analysis software such as Zen software, a one-component model is selected, in which the diffusion time of the complex is measured. FCCS uses two fluorescently-labeled capturing agents for VWF, making it possible to perform a more accurate analysis.

The following will describe specific functions. Any of the functions described below is known (see, for example, Krishevsky O. and Bonnet G., Rep. Prog. Phys. vol. 65, no. 2, 251, 2002). A fitting function $G(\tau)$ for determining a one-component three-dimensional diffusion correlation curve is expressed below by Eq. 1A. A fitting function $G(\tau)$ for determining a two-component three-dimensional diffusion correlation curve is expressed below by Eq. 1B. Both of these functions represent a translational diffusion process. When the translational diffusion time is calculated from a cross-correlation curve, a fitting model can be used as in Eq. 1A or Eq. 1B described below.

[Math 1]

$$G(\tau) = \frac{1}{\bar{N}} \left(1 + \frac{\tau}{\tau_D}\right)^{-1} \left(1 + \frac{\tau}{\omega^2 \tau_D}\right)^{-1/2} \qquad \text{Eq. 1A}$$

[Math 2]

$$G(\tau) = \frac{Q_1^2 \bar{N}_1}{\left(Q_1 \bar{N}_1 + Q_2 \bar{N}_2\right)^2} \left(1 + \frac{\tau}{\tau_{D1}}\right)^{-1} \left(1 + \frac{\tau}{\omega^2 \tau_{D1}}\right)^{-1/2}$$
$$+ \frac{Q_2^2 \bar{N}_2}{\left(Q_1 \bar{N}_1 + Q_2 \bar{N}_2\right)^2} \left(1 + \frac{\tau}{\tau_{D2}}\right)^{-1} \left(1 + \frac{\tau}{\omega^2 \tau_{D2}}\right)^{-1/2} \qquad \text{Eq. 1B}$$

In Eq. 1A, N denotes the number of molecules of a given component in a measurement region (in the formula, N.bar denotes the average number of molecules of a given component in a measurement region). $\tau_D$ denotes diffusion time (in $\mu$s). $\tau$ denotes correlation time. $\omega$ denotes the ratio ($w_z/w_{xy}$) between the radius of a laser focal volume (observation region) ($w_{xy}$) and half its length on the vertical axis ($w_z$). In Eq. 1B, $N_1$ denotes the number of molecules of a first component in a measurement region, and $N_2$ the number of molecules of a second component in the measurement region (in the formula, $N_1$.bar denotes the average number of molecules of a first component in a measurement region, and $N_2$.bar the average number of molecules of a second component in the measurement region). $Q_1$ denotes the quantum yield of the first component, and $Q_2$ the quantum yield of the second component. $\tau_{D1}$ denotes the diffusion time (in $\mu$s) of the first component, and $\tau_{D2}$ the diffusion time (in $\mu$s) of the second component. $\omega$ is preferably assigned at the beginning of the measurement, based on known diffusion times obtained from a mixture of 10 nM Alexa Fluor® 488 and 10 nM Alexa Fluor® 647 which are used as references.

The number of molecules of a given component is a value obtained from the value of $G(\tau)$ at $\tau = 0$. The diffusion time is denoted by the correlation time $\tau$ at which, when the value of $G(\tau)$ at $\tau = 0$ is set as a value at the initial point, the value at the initial point decreases by half. Specifically, if $G(\tau)$ gives a value of "a" when the correlation time $\tau$ is 0, that is, if $G(0) = a$, then the diffusion time means the correlation time $\tau$ at which $G(\tau)$ equals to a x 1/2. More specifically, in the correlation function $G(\tau)$, if the value of $G(0)$ is converted into 1, then the diffusion time is the correlation time $\tau$ at which $G(\tau)$ equals to 0.5. Thus, the correlation function $G(\tau)$ may be normalized so that it has a maximum value of "1" and a minimum value of "0." When the correlation function $G(\tau)$ is normalized, a combination of its maximum and minimum values can be set as appropriate. For example, such a combination may be one in which the maximum value is "2" and the minimum value is "1," or one in which the maximum value is "100" and the minimum value is "0."

In order to calculate the triplet state transition time, a fitting model may be used as in Eq. 2 described below. Eq. 2 represents a triplet state transition. In Eq. 2, T denotes triplet state amplitude. $\tau_T$ denotes the triplet state decay time of the fluorophore of a fluorescent dye.

[Math 3]

$$G(\tau) = \left(1 + \frac{T}{1-T} e^{\frac{-\tau}{\tau_T}}\right) \qquad \text{Eq. 2}$$

For an autocorrelation function, a fitting model may be used as in Eq. 3 described below, including both the translational diffusion time and the triplet state transition time of a fluorescent dye (T $\times$ 3D or T $\times$ {3D + 3D}). In Eq. 3, i represents the number of independent components.

[Math 4]

$$G(\tau) = \prod_i G_i(\tau) \qquad \text{Eq. 3}$$

The diffusion time is also a parameter that varies depending on the size of a fluorescently-labeled molecule. The information on the size of a fluorescently-labeled VWF may be a value obtained based on the diffusion time of the VWF. The value obtained based on the diffusion time of the fluorescently-labeled VWF may be a value calculated using the diffusion time. For example, the diffusion coefficient (D) is calculated from the diffusion time ($\tau_D$) and the radius of the observation region ($w_{xy}$) by using the formula: D = $w_{xy}^2/4\tau_D$. When it is assumed that a given fluorescently-labeled molecule is a spherical particle, the hydrodynamic radius ($R_H$) of the molecule is calculated from the formula: $R_H = k_B T/6\pi\eta D$. Here, $k_B$ is the Boltzmann constant, T is temperature (in K), and $\eta$ is the viscosity (in Pa·s) of a measurement sample. Using these equations and the diffusion time of the fluorescently-labeled VWF, the hydrodynamic radius can be obtained as information on the size of the VWF.

The value obtained based on the diffusion time of the fluorescently-labeled VWF may be a molecular size that is obtained by applying the diffusion time of the VWF to a standard curve, which is constructed from the diffusion times of VWFs with known molecular sizes. The molecular size is not required to be a quantitative value, such as a molecular weight or a volume, and may be a relative value. It is known that VWF is sheared by blood flow and forms into fragments with smaller molecular sizes. As shown in Example 4 described later, a plurality of measurement samples comprising VWF at different ratios of a large-molecular-weight VWF multimers can be prepared, for example, by agitating of normal human plasma, depending on the agitating time. The ratio of a large-molecular-weight VWF multimers can be determined, for example, by examining the composition of VWF multimers in a agitated and non-agitated sample by SDS-gel electrophoresis and Western blotting using an anti-VWF antibody, as described above. Specifically, an image of a Western blot is analyzed to calculate the ratio of the amount of a large-molecular-weight VWF multimer contained in the agitated sample to that in the non-agitated sample. The analysis and calculation of the ratio of such a VWF multimer are known and described, for example, in Boender J. et al., Hemasphere, 5(3): e542, 2021.

In a preferred embodiment, a plurality of reference samples having known ratios of a given large-molecular-weight VWF multimer is used to construct a standard curve by denaturing these reference samples with urea, fluorescently labeling the denatured samples with a fluorescently-labeled capturing agent or agents, and obtaining their diffusion times by FCS or FCCS, respectively, in a similar way as in a biological sample, and then plotting the diffusion times and the ratios of the large-molecular-weight VWF multimer. After that, the diffusion time for VWF contained in the biological sample is obtained and applied to the standard curve, whereby the ratio of the large-molecular-weight VWF multimer for the biological sample can be obtained as information on the size of the VWF.

An advantage of an information obtaining method according to the present invention is that as described above, the method reduces the influence of the concentration of VWF in a biological sample or a measurement sample and obtains information on the size of the VWF. A further advantage is that an information obtaining method according to the present invention requires a shorter time to obtain a desired result, relative to a combination of SDS-gel electrophoresis and Western blotting, which are conventional methods. The combination of SDS-gel electrophoresis and Western blotting usually requires one or two days to obtain information on the size of the VWF. An information obtaining method according to the present invention, however, can obtain the information in the order of 30 minutes to 1 hour.

A further related aspect of the present invention relates to a method for preparing a measurement sample for use in measurement by FCS or FCCS (which is also referred to hereinafter as a "preparation method according to the present invention"). According to the preparation method according to the present invention, a measurement sample suitable for obtaining the diffusion time of fluorescently-labeled VWF can be prepared from a biological sample comprising VWF. Specifically, VWF contained in a biological sample is first denatured with urea. Details of this urea denaturing are the same as those described for an information obtaining method according to the present invention.

The denatured VWF is then fluorescently labeled using a fluorescently-labeled capturing agent or agents. Details of the fluorescently-labeled capturing agent or agents and the fluorescent labelling thereof are the same as those described for an information obtaining method according to the present invention. The type of the capturing agent or agents is a polyclonal antibody, a plurality of monoclonal antibodies that bind to epitopes different from each other, a plurality of aptamers that bind to epitopes different from each other, and a combination thereof, as in an information obtaining method according to the present invention.

When a measurement sample for measurement by FCS is prepared, one capturing agent is used that has been labeled with one fluorescent substance. When a measurement sample for measurement by FCCS is prepared, two capturing agents are used that have been labeled with each of two fluorescent substances. This means that a first fluorescently-labeled capturing agent and a second fluorescently-labeled capturing agent are used as the fluorescently-labeled capturing agents. As described above, it is preferable that the second fluorescent substance in the second fluorescently-labeled capturing agent has a maximum fluorescence emission in a wavelength range different from that of the first fluorescent substance in the first fluorescently-labeled capturing agent. Details of the first and second fluorescently-labeled capturing agents and the respective fluorescent substances thereof are the same as those described for an information obtaining method according to the present invention.

A further related aspect of the present invention relates to the use of a reagent kit in an information obtaining method according to the present invention and the preparation method according to the present invention as described above (which is also referred to hereinafter as a "reagent kit according to the present invention"). A reagent kit used in a method according to the present invention comprises a first reagent comprising urea and a second reagent comprising a fluorescently-labeled capturing agent. Details of the fluorescently-labeled capturing agent are the same as those described for an information obtaining method according to the present invention.

The urea in the first reagent may be in solid or in solution. In a preferred embodiment, the first reagent preferably comprises a urea solution. The solvent of said solution can be selected from the aqueous solvents described in the description of an information obtaining method according to the present invention. There is no particular limitation on the concentration of urea in the first reagent. For the concentration of urea in the first reagent, it would be sufficient if the final concentration of urea in a mixture of a biological sample and the first reagent is, for example, not less than 0.5 M and not more than 1.75 M. Specifically, the concentration of urea in the first reagent can be not less than 1 M and not more than 8 M, preferably not less than 1.5 M and not more than 6 M, and more preferably not less than 2 M and not more than 4 M.

When information on the size of VWF is obtained by FCS in an information obtaining method according to the present invention, or when a measurement sample for measurement by FCS is prepared in a preparation method according to the present invention, the second reagent comprises a capturing agent that has been labeled with one fluorescent substance. When information on the size of VWF is obtained by FCCS in an information obtaining method according to the present invention, or when a measurement sample for measurement by FCCS is prepared in a preparation method according to the present invention, the second reagent comprises a first fluorescently-labeled capturing agent and a second fluorescently-labeled capturing agent. Alternatively, a first fluorescently-labeled capturing agent and a second fluorescently-labeled capturing agent may each be comprised in different reagents. In this case, the reagent kit comprises a first reagent comprising urea, a second reagent comprising a first fluorescently-labeled capturing agent, and a third reagent comprising a second fluorescently-labeled capturing agent. Details of the first and second fluorescently labeled capturing agents and the respective fluorescent substances thereof are the same as those described for an information obtaining method according to the present invention.

The fluorescently-labeled capturing agent(s) in the second reagent may be in solid (for example, powder form, crystal form, freeze-dried product, etc.) or in liquid (for example, solution, suspension, emulsion, etc.). In a preferred embodiment, the second reagent preferably comprises a solution of the fluorescently-labeled capturing agent. The solvent of said solution can be selected from the aqueous solvents described in the description of an information obtaining method according to the present invention. To the aqueous medium, a stabilizer such as bovine serum albumin (BSA) or casein may be added as necessary. There is no particular limitation on the concentration of the fluorescently-labeled capturing agent(s) in the second reagent. For the concentration of the fluorescently-labeled capturing agent in the second reagent, in cases when it is an antibody, it would be sufficient if the final concentration of the antibody in a measurement sample is, for example, not less than 1 nM and not more than 100 nM. Specifically, the concentration of the fluorescently-labeled capturing agent in the second reagent can be not less than 2 nM and not more than 200 nM, preferably not less than 5 nM and not more than 150 nM, and more preferably not less than 10 nM and not more than 100 nM. In cases when the first fluorescently-labeled capturing agent and the second fluorescently-labeled capturing agent are antibodies, it would be sufficient if the final concentration of each of these antibodies in the reagent is within the above-described range.

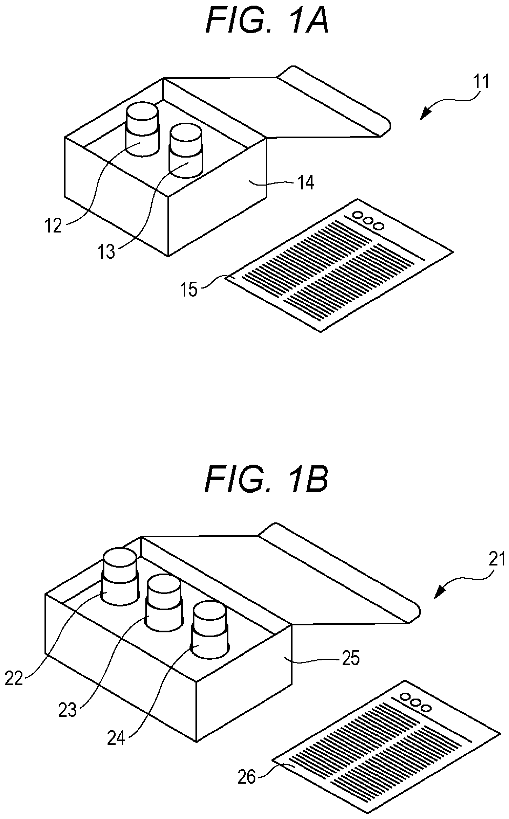

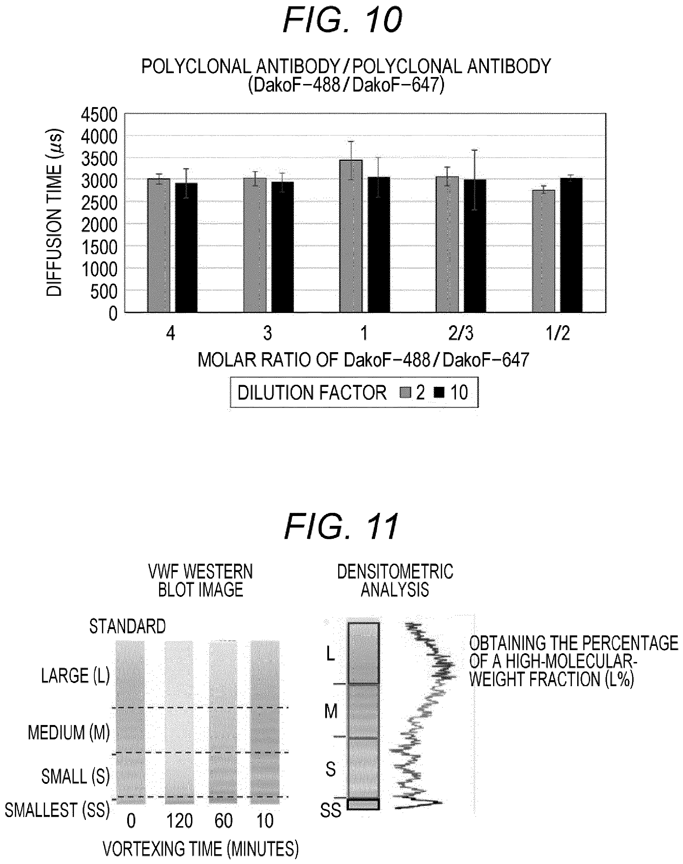

A reagent kit used in a method according to the present invention may be packaged in a box comprising containers each containing the reagents, thereby to be provided for a user. The box may include a package insert. The package insert may describe the compositions of and methods for use and storage of the respective reagents, and others. Fig. 1A represents an example of a reagent kit used in a method according to the present invention. Referring to Fig. 1A, 11 represents a reagent kit used in a method according to the present invention; 12 represents a first container in which a first reagent comprising urea is contained; 13 represents a second container in which a second reagent comprising a fluorescently-labeled capturing agent is contained; 14 represents a packaging box; and 15 represents a package insert. The second container 13 may contain a second reagent comprising a first fluorescently-label capturing agent and a second fluorescently-labeled capturing agent.

Fig. 1B represents an example of a reagent kit used in a method according to the present invention in which a first fluorescently-labeled capturing agent and a second fluorescently-labeled capturing agent are contained in a second reagent and a third reagent, respectively. Referring to Fig. 1B, 21 represents a reagent kit used in a method according to the present invention; 22 represents a first container in which a first reagent comprising urea is contained; 23 represents a second container in which a second reagent comprising a first fluorescently-labeled capturing agent is contained; 24 represents a third container in which a third reagent comprising a second fluorescently-labeled capturing agent is contained; 25 represents a packaging box; and 26 represents a package insert.

A further related aspect of the present invention relates to use of urea and a capturing agent that comprises a fluorescent substance and binds to urea-denatured VWF. These substances are used for the production of a reagent kit for obtaining information on the size of VWF by FCS, or a reagent kit for preparing a measurement sample for obtaining such information. The capturing agent comprises a polyclonal antibody, or a plurality of monoclonal antibodies or aptamers that bind to epitopes different from each other. Details of the urea denaturing, the fluorescent substance, and the capturing agent are the same as those described for an information obtaining method according to the present invention.

A further aspect of the present invention relates to use of urea, a first capturing agent that comprises a first fluorescent substance and binds to urea-denatured VWF, and a second capturing agent that comprises a second fluorescent substance and binds to the urea-denatured VWF. These substances are used for the production of a reagent kit for obtaining information on the size of VWF by FCCS, or a reagent kit for preparing a measurement sample for obtaining such information. The first capturing agent that comprises the first fluorescent substance and binds to urea-denatured VWF comprises a polyclonal antibody, or a plurality of monoclonal antibodies or aptamers that bind to epitopes different from each other. Details of the urea denaturing, the respective fluorescent substances, and the respective capturing agents are the same as those described for an information obtaining method according to the present invention.

The present invention is now described in more detail by way of examples, but is not limited thereto.

EXAMPLES

Reference Example

When a plurality of measurement samples comprising VWF at different concentrations was measured by FCCS, it was investigated whether there was a difference in the diffusion times of VWF obtained from these measurement samples. In this Reference Example, a comparison was made between measurements of VWF that was subjected to direct and indirect labeling with fluorescent dyes.

(1) Biological sample

Human plasma-derived VWF protein (Merck) was labeled with Alexa Fluor® 488 and Alexa Fluor® 647 (Thermo Fisher Scientific) by amine coupling to obtain VWF having the two fluorescent dyes covalently bonded thereto (hereinafter referred to as "488-VWF-647"). The 488-VWF-647 was added to PBS containing 1% (w/v) BSA (pH 7.4) (hereinafter referred to as "1% BSA-PBS") to a concentration of 25 nM in terms of VWF, thereby to prepare a 488-VWF-647 solution. A VWF solution was also prepared by adding human plasma-derived VWF protein to 1% BSA-PBS to a concentration of 25 nM.

(2) Reagent comprising fluorescently-labeled antibodies

NMC4 Fab and antibody 2F2A9 (BD Biosciences) were used as monoclonal antibodies that bind to VWF. These are antibodies that bind to epitopes different from each other. NMC4 Fab was prepared by a known genetic recombination, based on a published amino acid sequence. Specifically, the light chain and the heavy chain of NMC4 were first expressed in Expi 293 cells, and then the culture supernatant was collected. The NMC4 Fab in the culture supernatant was purified by gel filtration and concentrated by a centrifugal concentrator. The NMC4 Fab obtained was labeled with Alexa Fluor® 647 by amine coupling to obtain Alexa Fluor 647-labeled NMC4 Fab (hereinafter, referred to as "NMC4-647"). Antibody 2F2A9 was labeled with Alexa Fluor® 488 by a maleimide method, and the unbound fluorescent dye was removed by a desalting column to obtain an Alexa Fluor 488-labeled 2F2A9 antibody (hereinafter, referred to as "2F2A9-488"). Each of these antibodies were added to 1% BSA-PBS to prepare reagent 1 comprising 2F2A9-488 (50 nM) and NMC4-647 (25 nM).

(3) Preparation of measurement samples

(3.1) Measurement samples comprising 488-VWF-647

The 488-VWF-647 solution (50 μL) and 1% BSA-PBS (50 μL) were mixed to prepare a measurement sample with a dilution factor of 2 times. The 488-VWF-647 solution (10 μL) and 1% BSA-PBS (90 μL) were mixed to prepare a measurement sample with a dilution factor of 10 times. The 488-VWF-647 solution (5 μL) and 1% BSA-PBS (95 μL) were mixed to prepare a measurement sample with a dilution factor of 20 times.

(3.2) Measurement samples comprising VWF

The VWF solution (50 μL) and the reagent 1 (50 μL) were mixed and incubated at 37°C for 5 minutes to prepare a measurement sample with a dilution factor of 2 times. The VWF solution (10 μL), 1% BSA-PBS (40 μL), and the reagent 1 (50 μL) were mixed and incubated at 37°C for 5 minutes to prepare a measurement sample with a dilution factor of 10 times. The VWF solution (5 μL), 1% BSA-PBS (45 μL), and the reagent 1 (50 μL) were mixed and incubated in dark at room temperature for 5 minutes to prepare a measurement sample with a dilution factor of 20 times.

(4) Measurement and analysis

Each of these measurement samples (30 μL) was added to a well of a 384 well glass-bottom plate (Sigma-Aldrich) and measured with a Carl Zeiss LSM 710 confocal laser microscope (Zeiss). The measurement was made in triplicate per measurement sample. Alexa Fluor® 488 was excited with a 488 nm laser beam, and Alexa Fluor® 647 with a 639 nm laser beam. The power of each of the 488-nm and 639-nm laser beams was 1 to 10 μW. The fluorescence intensity

(kHz) was continuously measured with a measurement time of 10 seconds to 15 seconds per measurement for each well. The measurement was carried out 10 to 15 times for each well. Fitting of an average correlation function for these 10 to 15 measurements was performed. The analysis was performed using Zen software (Zeiss). The raw data obtained were processed to eliminate a measurement value representing a baseline drift or a burst (a fluorescence signal having three times or more brightness than an average fluorescence brightness). The cross-correlation curve used in FCCS was fitted using a one-component three-dimensional translational diffusion model (3D). When the fitting of the cross-correlation function was done in a one-component model, the component was an immunocomplex between the first and second fluorescently-labeled antibodies and VWF. A one-component model was selected on the Zen software. The diffusion time of the immunocomplex was obtained for each of the measurement samples, based on the signals from the first and second fluorescent substances.

(5) Results

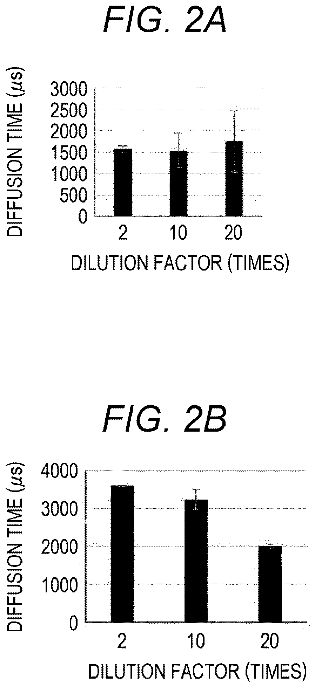

Fig. 2A shows the diffusion times of the respective 488-VWF-647-containing measurement samples. Fig. 2B shows the diffusion times of the respective VWF-containing measurement samples. As shown in Fig. 2A, the diffusion times obtained from the 488-VWF-647-containing measurement samples were almost constant regardless of dilution factors of the biological samples. This indicates that when VWF was subjected to direct labeling with the fluorescent dyes, the molecular size of the VWF obtained by FCCS did not depend on the concentration of the VWF. As shown in Fig. 2B, on the other hand, the higher the dilution factor of the biological sample, the lower the diffusion time obtained from the VWF-containing measurement samples became. This indicates that when VWF was subjected to indirect labeling with the fluorescently-labeled antibodies, the molecular size of the VWF obtained by FCCS depended on the concentration of the VWF.

The present inventors have considered that these results are due to insufficient exposure of epitopes on non-denatured VWF and a small number of molecules of the fluorescently-labeled antibody binding to VWF. Insufficient exposure of epitopes on VWF can affect the number of molecules of the antibody that are to bind to VWF. A small number of molecules of the antibody binding to VWF is indicative that the dissociation of the antibody from the VWF can result in a great influence on the molecular size thereof. Since the lower the concentration of VWF in a measurement sample, the higher the frequency at which the antibody dissociates from VWF becomes, it is assumed that the average size of the complex between the antibody and VWF decreases. The present inventors have considered that in order to reduce the influence of VWF concentration, that is, the influence due to the dissociation of VWF and the antibody, it is necessary to increase the number of bound antibody molecules per molecule of VWF. The present inventors have studied denaturing of VWF with a protein denaturing agent and changing of the type of fluorescently-labeled antibody.

Example 1

The present inventors investigated whether the effect of reducing the influence of VWF concentration can be obtained by denaturing VWF and by changing the above-described fluorescently-labeled antibodies.

(1) Biological sample

Use was made of standard human plasma for coagulation test (Sysmex Corporation), as a biological sample.

(2) Reagents

(2.1) Denaturing agent

Urea (9 g, FUJIFILM Wako Pure Chemical Corporation) was dissolved in 1% BSA-PBS to make a 3 M urea solution of 50 mL.

(2.2) Fluorescent substances

Use was made of Alexa Fluor® 488 (Thermo Fisher Scientific), as a first fluorescent substance. Use was made of Alexa Fluor® 647 (Thermo Fisher Scientific), as a second fluorescent substance. Hereinafter, an antibody labeled with Alexa Fluor® 488 is also referred to as a "first fluorescently-labeled antibody." An antibody labeled with Alexa Fluor® 647 is also referred to as a "second fluorescently-labeled antibody."

(2.3) Reagent comprising fluorescently-labeled antibodies

(i) Preparation of fluorescently-labeled monoclonal antibodies

In addition to NMC4 Fab and antibody 2F2A9 used in the above-described Reference Examples, antibodies VWF 635 (Novus Biologicals) and SPM 577 (Novus Biologicals) were used as a monoclonal antibody that binds to VWF. Antibodies VWF 635 and SPM 577 are ones that bind to epitopes different from each other. NMC4-647 and 2F2A9-488 were prepared from NMC4 Fab and antibody 2F2A9, respectively, in a similar way as in the above-described Reference Example. Antibodies VWF 635 and SPM 577 were each labeled with Alexa Fluor® 488 by amine coupling to obtain an Alexa Fluor 488-labeled VWF 635 antibody (hereinafter, referred to as "VWF-488") and an Alexa Fluor 488-labeled SPM 577 antibody (hereinafter, referred to as "SPM-488"), respectively.

(ii) Preparation of fluorescently-labeled polyclonal antibodies

Polyclonal anti VWF IgG (Dako A/S) was used as a polyclonal antibody that binds to VWF. This polyclonal antibody was fragmented by pepsin digestion, and Polyclonal anti VWF F(ab')2 was purified and obtained by gel filtration. The resulting F(ab')2 was labeled with Alexa Fluor®488 or with Alexa Fluor® 647 by amine coupling to obtain an Alexa Fluor 488-labeled Polyclonal anti VWF F(ab')2 (hereinafter, referred to as "DakoF-488") and an Alexa Fluor 647-labeled Polyclonal anti VWF F(ab')2 (hereinafter, referred to as "DakoF-647"), respectively.

(iii) Preparation of reagents comprising fluorescently-labeled antibodies

Reagents 1 to 3 were prepared by combinations of the above-described fluorescently-labeled antibodies. Reagent 1 was the same reagent as in the above-described Reference Example, that is, a reagent comprising 2F2A9-488 (50 nM) and NMC4-647 (25 nM). Reagent 2 was a reagent comprising DakoF-488 (50 nM) and NMC4-647 (25 nM). Reagent 3 was a reagent comprising VWF-488 (25 nM), SPM-488 (25 nM), and NMC4-647 (25 nM). The compositions of these reagents are shown in Table 1. In the table, "488-labeled antibody" and "647-labeled antibody" denote an antibody labeled with Alexa Fluor® 488 and an antibody labeled with Alexa Fluor® 647, respectively. In the table, the concentration of each of the antibodies indicates its final concentration in the reagent. 1% BSA-PBS was used as the solvent of reagents 1 to 3.

[Table 1]

| | 488-Labeled antibody | Concentration (nM) | 647-Labeled antibody | Concentration (nM) |
|---|---|---|---|---|
| Reagent 1 | 2F2A9-488 | 50 | NMC4-647 | 25 |
| Reagent 2 | DakoF-488 | 50 | NMC4-647 | 25 |
| Reagent 3 | VWF-488 | 25 | NMC4-647 | 25 |
| | SPM-488 | 25 | | |

(3) Preparation of measurement samples

A plurality of measurement samples with different concentrations of VWF was prepared by dilution of the biological sample. A measurement sample with a dilution factor of 2 times was prepared as follows. The biological sample (17 μL) and 1% BSA-PBS (17 μL) were mixed, thereby resulting in a 2-times dilution of the biological sample. To the diluted biological sample, a 3 M urea solution (17 μL) or 1% BSA-PBS (17 μL) was added, and the mixture was incubated at 37°C for 15 minutes. The sample after the addition of the urea solution had a final concentration of urea of 1 M. Reagent 1 (50 μL), 2 (50 μL), or 3 (50 μL) was then added to each of the incubated samples, and the mixture was incubated in dark at room temperature for 5 minutes to obtain a measurement sample. A measurement sample with a dilution factor of 10 times was prepared in a similar way as the measurement sample with a dilution factor of 2 times except that the biological sample (3.4 μL) and 1% BSA-PBS (30.6 μL) were mixed. The sample after the addition of the reagent solution had a final concentration of urea of 0.495 M.

(4) Measurement and analysis

Each of these measurement samples (30 μL) was added to a well of a 384 well glass-bottom plate (Sigma-Aldrich) and measured with a Carl Zeiss LSM 710 confocal laser microscope (Zeiss). Alexa Fluor® 488 was excited with a 488 nm laser beam, and Alexa Fluor® 647 with a 639 nm laser beam. The power of each of the 488-nm and 639-nm laser beams was 1 to 10 μW. The fluorescence intensity (kHz) was continuously measured with a measurement time of 10 seconds to 15 seconds per measurement for each well. The measurement was carried out 10 to 15 times. Fitting of an average correlation function for these 10 to 15 measurements was performed. The analysis was performed using Zen software (Zeiss). The raw data obtained were processed to eliminate a measurement value representing a baseline drift or a burst (a fluorescence signal having three times or more brightness than an average fluorescence brightness). The cross-correlation curve used in FCCS was fitted using a one-component three-dimensional translational diffusion model (3D). The autocorrelation curve used in FCS was fitted using a one-component or two-component three-dimensional translational diffusion + triplet state model (T × 3D or T × {3D + 3D}).

(i) Obtaining diffusion time by FCS

When the fitting of the autocorrelation function was done in a one-component model, the component was an immunocomplex between the first or second fluorescently-labeled antibody and VWF. A one-component model was selected on the Zen software. The diffusion time of the immunocomplex was obtained for each of the measurement samples, based on the signal from the first or second fluorescent substance.

When the fitting of the autocorrelation function was done in a two-component model, a first component was an unreacted first or second fluorescently-labeled antibody. A second component was an immunocomplex between the first or second fluorescently-labeled antibody and VWF. A one-component model was first selected on the Zen software. The diffusion time of the first component ($Tp_{488}$ or $Tp_{647}$) was obtained by adding each of the fluorescently-labeled antibodies to a VWF-free sample (a mixed solution of 1% BSA-PBS (34 μL) and a 3 M urea solution (17 μL)), and measuring the mixture. After that, a two-component model was selected on the Zen software. The diffusion time of the first component was fixed to the previously-measured diffusion time of the first component ($Tp_{488}$ or $Tp_{647}$). The diffusion time of the second component was obtained for each of the measurement samples, based on the signal from the first or second fluorescent substance.

(ii) Obtaining diffusion time by FCCS

When the fitting of the cross-correlation function was done in a one-component model, the component was an immunocomplex between the first and second fluorescently-labeled antibodies and VWF. A one-component model was selected on the Zen software. The diffusion time of the immunocomplex was obtained for each of the measurement samples, based on the signals from the first and second fluorescent substances.

(5) Results

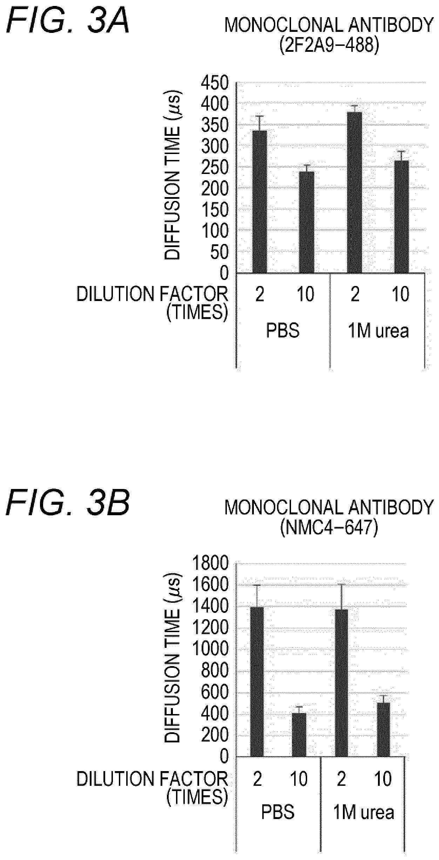

Figs. 3A, 3B, and 4 to 8 show the diffusion times obtained by FCS or FCCS for the measurement samples. In each of the figures, "PBS" denotes a measurement sample to which 1% BSA-PBS was added instead of the 3 M urea solution. "1 M urea" denotes a measurement sample after the addition of the 3 M urea solution. 1 M was the final concentration of urea when the 3 M urea solution was added to the diluted biological sample. Table 2 shows the correspondence between these figures and the reagents, the measurement wavelengths, the fluorescently-labeled antibodies to be detected, and the measurement methods that were used.

[Table 2]

| Figure | Reagent | Measured wavelength (nm) | Fluorescently-labeled antibody/antibodies | Measurement method |
|---|---|---|---|---|
| **3A** | Reagent 1 | **488** | **2F2A9-488** | **FCS** |
| **3B** | | **647** | **NMC-647** | |
| **4** | Reagent 2 | **488** | **DakoF-488** | |
| **5** | Reagent 3 | | VWF-488 and SPM-488 | |
| **6** | Reagent 1 | **488/647** | **2F2A9-488/NMC-647** | **FCCS** |
| **7** | Reagent 2 | **488/647** | **DakoF-488/NMC-647** | |
| **8** | Reagent 3 | **488/647** | VWF-488 and SPM-488 / NMC 4-647 | |

Figs. 3A and 3B show the diffusion times obtained by FCS (at a measurement wavelength of 488 nm or 647 nm)

15

using reagent 1. Although reagent 1 comprised two fluorescently-labeled antibodies, 2F2A9-488 and NMC4-647, the measurements were carried out at a measurement wavelength of either 488 nm or 647 nm, and thus were substantially the same as those by FCS using one fluorescently-labeled antibody. As shown in Figs. 3A and 3B, the diffusion time of the 10-times diluted measurement sample was shorter than that of the 2-times diluted measurement sample, regardless of with or without urea denaturation. Reagent 1 was the same as the reagent used in the above-described Reference Example. It is suggested that the urea denaturation alone does not make it possible to reduce the influence on measurement results by FCS due to the concentration of VWF contained in a measurement sample.

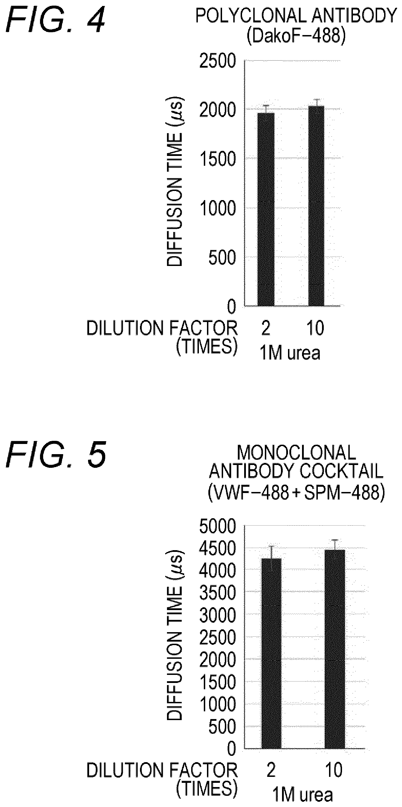

Figs. 4 and 5 show the diffusion times obtained by FCS (at a measurement wavelength of 488 nm) using reagent 2 or 3, respectively. Since reagent 2 comprises a fluorescently-labeled antibody derived from a polyclonal antibody (DakoF-488), and reagent 3 comprises two fluorescently-labeled antibodies derived from two monoclonal antibodies that bind to epitopes different from each other (VWF-488 and SPM-488), it was expected that the number of bound antibody molecules per molecule of VWF was increased as compared to the case when reagent 1 was used. As shown in Figs. 4 and 5, when urea denaturation was performed, there was little difference in the diffusion times of the 2-times and 10-times diluted measurement samples. These results suggest that the influence of the concentration of VWF contained in a measurement sample can be reduced by denaturing, with urea, VWF contained in a biological sample, and labeling, with one fluorescent substance, a polyclonal antibody, or two or more monoclonal antibodies that bind to epitopes different from each other, and using the fluorescently-labeled polyclonal antibody or monoclonal antibodies in FCS. Although the denaturing action of urea extends not only to the VWF, but also to the fluorescently-labeled antibody(s), the above-described results also suggest that a measurement sample having a final concentration of urea of about 0.5 M causes no influence on measurement.

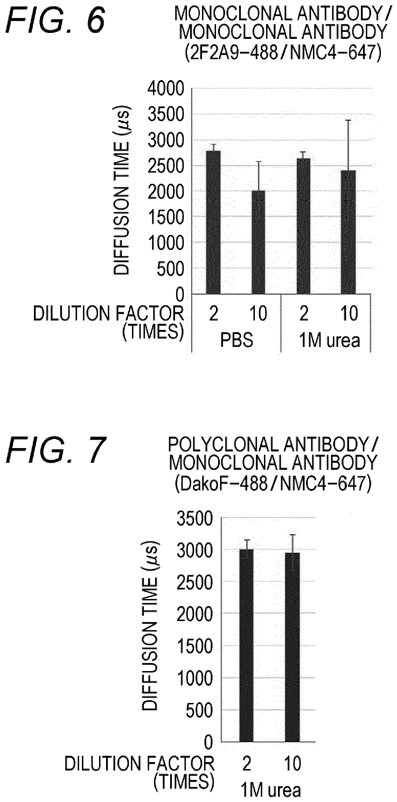

Fig. 6 shows the diffusion times obtained by FCCS (at measurement wavelengths of 488 nm and 647 nm) using reagent 1. As shown in Fig. 6, the diffusion time of the 10-times diluted measurement sample was shorter than that of the 2-times diluted measurement sample, in the case without urea denaturation. The samples with urea denaturation were found to tend to reduce the difference in the diffusion times between the measurement samples having different dilution factors, relative to the samples without urea denaturation. These findings suggest that the urea denaturation alone does not make it possible to achieve a sufficient reduction of the influence on measurement results by FCCS due to the concentration of VWF contained in a measurement sample.

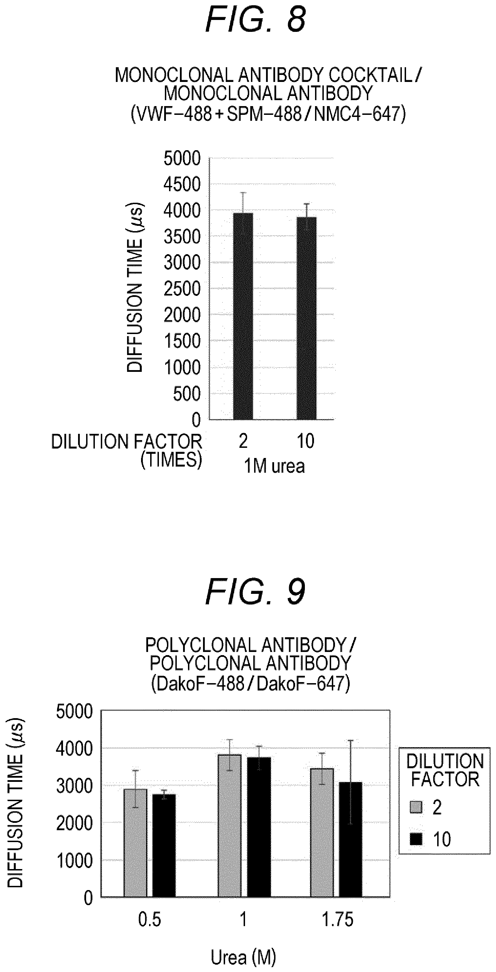

Figs. 7 and 8 show the diffusion times obtained by FCCS using reagent 2 or 3, respectively. As shown in Figs. 7 and 8, there was little difference in the diffusion times of the 2-times and 10-times diluted measurement samples with urea denaturation. FCCS uses two fluorescent substances. These results suggest that it is useful in reducing the influence of VWF concentration to fluorescently label, with either of two fluorescent substances, a polyclonal antibody, or two or more monoclonal antibodies that bind to epitopes different from each other, and to use the fluorescently-labeled polyclonal antibody or monoclonal antibodies in FCCS. It is suggested that it is also useful to denature VWF with urea. The above-described results also suggest that a measurement sample having a final concentration of urea of about 0.5 M causes no influence on measurement.

Example 2

An investigation was made of the concentration of urea during the denaturing of VWF contained in a biological sample. In Example 2, the diffusion time was obtained by FCCS using a combination of polyclonal antibodies labeled with each of two fluorescent substances.

(1) Biological sample and reagents

Use was made of the same standard human plasma for coagulation test as in Example 1, as a biological sample. Use was made of same 3M urea solution as in Example 1, as a denaturing agent. 4.5 g or 15.75 g of Urea (FUJIFILM Wako Pure Chemical Corporation) was dissolved in 1% BSA-PBS to make a solution of 50 mL to prepare 1.5 M and 5.25 M urea solutions, respectively. Use was made of DakoF-488 and DakoF-647 prepared in Example 1, as fluorescently-labeled antibodies. As a reagent comprising these fluorescently-labeled antibodies, reagent 4 was prepared which comprises DakoF-488 (50 nM) and DakoF-647 (25 nM). For the solvent of reagent 4, 1% BSA-PBS was used.

(2) Preparation of measurement samples

A plurality of measurement samples with different concentrations of VWF and urea was prepared. A measurement sample with a dilution factor of 2 times was prepared as follows. The biological sample (17 μL) and 1% BSA-PBS (17 μL) were mixed, thereby resulting in a 2-times dilution of the biological sample. To the diluted biological sample, the 1.5 M, 3 M, or 5.25 M urea solution (17 μL) was added, and the mixture was incubated at 37°C for 15 minutes. The resulting samples had a final concentration of urea of 0.5 M, 1 M, or 1.75 M. To each of these, reagent 4 (50 μL) was then added, and the mixture was incubated in dark at room temperature for 5 minutes to obtain a measurement sample. A measurement sample with a dilution factor of 10 times was prepared in a similar way as the measurement sample with a dilution factor of 2 times except that the biological sample (3.4 μL) and 1% BSA-PBS (30.6 μL) were mixed. The resulting samples had a final concentration of urea of 0.224 M, 0.495 M, or 1.085 M.

(3) Measurement and analysis

In a similar way as in Example 1, the diffusion time was obtained by FCCS using an LSM 710 confocal laser microscope (Zeiss) and Zen software (Zeiss). The results are shown in Fig. 9. In the figure, the concentration of urea was the final concentration of urea when the 3 M urea solution was added to the diluted biological samples. Table 3 shows the correspondence between figure 9 and the reagents, the measurement wavelengths, the fluorescently-labeled antibodies to be detected, and the measurement method that were used.

[Table 3]

| Figure | Reagent | Measured wavelength (nm) | Fluorescently-labeled antibodies | Measurement method |
|---|---|---|---|---|
| 9 | Reagent 4 | 488/647 | DakoF-488/DakoF-647 | FCCS |

(4) Results

As shown in Fig. 9, there were not great differences in the diffusion times between the 2-times and 10-times diluted measurement samples when urea denaturation was performed at any of the concentrations of 0.5 M, 1 M, and 1.75 M. These results suggest that a biological sample can be denatured in the presence of urea at a concentration of not less than 0.5 M and not more than 1.75 M. It is also suggested that it is useful in reducing the influence of VWF concentration, to use in FCCS, a combination of polyclonal antibodies labeled with each of two fluorescent substances.

Example 3

An investigation was made of the mixing ratio of polyclonal antibodies labeled with each of two fluorescent substances.

(1) Biological sample and reagents

Use was made of the same standard human plasma for coagulation test and the same 3 M urea solution as in Example 1, as a biological sample and as a denaturing agent, respectively. Use was made of DakoF-488 and DakoF-647 prepared in Example 1, as fluorescently-labeled antibodies. Reagents 5 to 9 were prepared as reagents comprising these two fluorescently-labeled antibodies. The compositions of these reagents are shown in Table 4. In the table, the concentration of each of the two antibodies indicates its final concentration in the respective reagents. For the solvent of reagents 5 to 9, 1% BSA-PBS was used.

[Table 4]

| Reagent | DakoF-488 (nM) | DakoF-647 (nM) | Molar ratio (DakoF-488: DakoF-647) |
|---|---|---|---|
| Reagent 5 | 100 | 25 | 4:1 |
| Reagent 6 | 75 | 25 | 3:1 |
| Reagent 7 | 25 | 25 | 1:1 |
| Reagent 8 | 50 | 75 | 2:3 |
| Reagent 9 | 50 | 100 | 1:2 |

(2) Preparation of measurement samples

A measurement sample with a dilution factor of 2 times was prepared as follows. The biological sample (17 μL) and 1% BSA-PBS (17 μL) were mixed, thereby resulting in a 2-times dilution of the biological sample. To the diluted biological sample, the 3 M urea solution (17 μL) was added, and the mixture was incubated at 37°C for 15 minutes. The resulting samples had a final concentration of urea of 1 M. To each of these, 50 μL of reagent 5, 6, 7, 8, or 9 was then added, and the mixture was incubated in dark at room temperature for 5 minutes to obtain a measurement sample. A measurement sample with a dilution factor of 10 times was prepared in a similar way as the measurement sample with a dilution factor of 2 times except that the biological sample (3.4 μL) and 1% BSA-PBS (30.6 μL) were mixed. The resulting samples had a final concentration of urea of 0.495 M.

(3) Measurement and analysis

In a similar way as in Example 1, the diffusion time was obtained by FCCS using an LSM 710 confocal laser microscope (Zeiss) and Zen software (Zeiss). The results are shown in Fig. 10. In the figure, the molar ratio of the fluorescently-labeled antibodies is a value of the ratio of the final concentration of DakoF-488 to that of DakoF-647 in each of the reagents. Table 5 shows the correspondence between figure 10 and the reagents, the measurement wavelengths, the fluorescently-labeled antibodies to be detected, and the measurement method that were used.

[Table 5]

| Figure | Reagent | Measured wavelength (nm) | Fluorescently-labeled antibodies | Measurement method |
|---|---|---|---|---|
| **10** | Reagents 5 to 9 | **488/647** | **DakoF-488/DakoF-647** | **FCCS** |

(4) Results

As shown in Fig. 10, there were no great differences in the diffusion times between the 2-times and 10-times diluted measurement samples at any of the molar ratios of DakoF-488 to DakoF-647 in the reagents. These results suggest that it would be sufficient if the ratio of the concentration of a first polyclonal antibody comprising a first fluorescent substance relative to that of a second polyclonal antibody comprising a second fluorescent substance is not less than 0.5 and not more than 4 in terms of the molar ratio of these polyclonal antibodies.

Example 4

In Example 4, an investigation was made of whether it was possible to obtain diffusion times according to molecular sizes, by agitating standard human plasma for coagulation test, thereby to prepare a plurality of measurement samples comprising VWF with different molecular sizes and measuring them by FCS or FCCS.

(1) Biological sample

Use was made of standard human plasma for coagulation test (Sysmex Corporation), as a standard plasma sample. The standard plasma sample was divided into four aliquots. 3 of the 4 aliquots were each agitated on a vortex mixer for 10, 60, or 120 minutes before EDTA was added to a final concentration of 10 μg/mL. The molecular size of VWF contained in each of the samples was analyzed by SRL Co., Ltd. In this analysis, the composition of VWF multimers was determined by SDS-agarose gel electrophoresis and Western blotting using an anti-VWF antibody. Fig. 11 shows an image of a Western blot of the respective samples. In the figure, "standard" denotes the standard plasma sample. As shown in Fig. 11, it is shown that the longer the stirring time, the smaller the molecular size of VWF became. This means that plasma samples comprising VWF with different molecular sizes were prepared by agitating the standard plasma sample. In this Western blot image, the VWF bands were classified into four fractions, Large, Medium, Small, and Smallest, according to their molecular sizes. Image J software (provided by NIH) was used to determine the concentrations of these bands in the image by densitometry for each of the plasma samples. Then, the percentage (L%) of the band concentration for the Large fraction relative to the total band concentration of all the fractions was calculated for every plasma sample. The LMW index (%) was calculated using the L% value of the standard plasma sample and those of the agitated samples, according to the following equation: For the analysis of VWF multimers based on Western blot images, reference was made to Boender J. et al., Hemasphere, 5(3): e542, 2021.

LMW index = [(L% value of agitated sample)/(L% value of standard plasma sample)] × 100

Based on the LMW index calculated for each of the samples, the standard plasma sample is referred to hereinafter as "100% SHP", the plasma sample agitated for 10 minutes "75% SHP", the plasma sample agitated for 60 minutes "50% SHP", and the plasma sample agitated for 120 minutes "25% SHP."

(2) Reagents

Use was made of the same 3M urea solution as in Example 1, as a denaturing agent. Use was made of the same reagent 4 as in Example 2, as a reagent comprising fluorescently-labeled antibodies.

(3) Preparation of measurement samples

A measurement sample with a dilution factor of 10 times was prepared as follows. 100% SHP (4 $\mu$L), 75% SHP (4 $\mu$L), 50% SHP (4 $\mu$L), or 25% SHP (4 $\mu$L) was mixed with 1% BSA-PBS (36 $\mu$L), thereby resulting in a 10-times dilution of each of these biological sample. To the diluted samples, the 3 M urea solution (20 $\mu$L) was added, and the mixture was incubated at 37°C for 15 minutes. The resulting samples had a final concentration of urea of 1 M. To each of these, reagent 4 (40 $\mu$L) was then added, and the mixture was incubated in dark at room temperature for 5 minutes to obtain a measurement sample. The resulting samples had a final concentration of urea of 0.6 M.

(4) Measurement and analysis

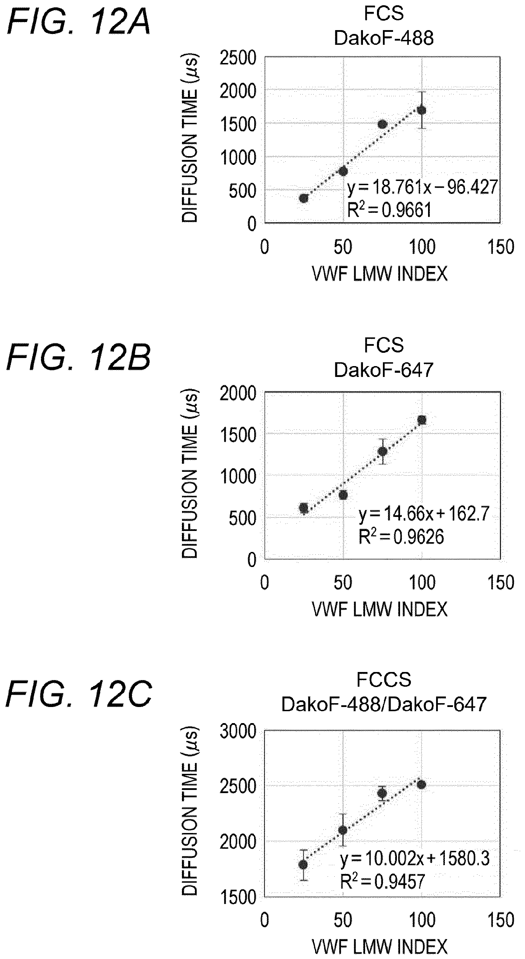

In a similar way as in Example 1, the diffusion time was obtained by FCS or FCCS using an LSM 710 confocal laser microscope (Zeiss) and Zen software (Zeiss). Figs. 12 A to C show graphs in which the diffusion times of the respective measurement samples are plotted. Table 6 shows the correspondence between these figures and the reagents, the measurement wavelengths, the fluorescently-labeled antibodies to be detected, and the measurement methods that were used.

[Table 6]

| Figure | Reagent | Measured wavelength (nm) | Fluorescently-labeled antibody/antibodies | Measurement method |
|--------|---------|--------------------------|-------------------------------------------|--------------------|
| **12A** | Reagent 4 | **488** | **DakoF-488** | **FCS** |
| **12B** | Reagent 4 | **647** | **DakoF-647** | **FCS** |
| **12C** | Reagent 4 | **488/647** | **DakoF-488/DakoF-647** | **FCCS** |

(5) Results

As shown in Figs. 12 A to C, it is shown that in any of the measurements, the diffusion time became longer as the LMW index increased. As also shown in Figs. 12 A to C, the coefficient of determination was 0.9 or more in any of the regression equations. These results suggest that it is possible to obtain the diffusion time according to the molecular size of VWF by measuring a measurement sample comprising urea-denatured VWF by FCS or FCCS using a fluorescently-labeled polyclonal antibody or antibodies, respectively.

Example 5

In this example, an investigation was made of whether urea denaturation and the use of fluorescently-labeled polyclonal antibodies reduced the influence of VWF concentration, by FCS and FCCS measurement of measurement samples with a dilution factor of 2 to 32 times prepared from 100% SHP and 50% SHP described above in Example 4.

(1) Biological sample and reagents

Use was made of the same 100% SHP and 50% SHP as in Example 4, as biological samples. Use was made of the same 3M urea solution as in Example 1, as a denaturing agent. Use was made of the same reagents 1 and 2 as in Example 1, as reagents comprising fluorescently-labeled antibodies.

(2) Preparation of measurement samples

A measurement sample with a dilution factor of 2 times was prepared by mixing 100% SHP (40 $\mu$L) and 1% BSA-PBS (40 $\mu$L). A 40 $\mu$L aliquot was taken from the resulting diluted plasma sample and mixed with 1% BSA-PBS (40 $\mu$L) to prepare a plasma sample with a dilution factor of 4 times. A similar procedure was repeated to prepare plasma samples with dilution factors of 8 times, 16 times, and 32 times. A similar dilution procedure for 50% SHP was carried out to prepare plasma samples with dilution factors of 2 times, 4 times, 8 times, 16 times, and 32 times. To each of the plasma samples obtained by dilution (34 μL), the 3 M urea solution (17 μL) was added, and the mixture was incubated at 37°C for 15 minutes. The resulting samples had a final concentration of urea of 1 M. To each of these, reagent 1 (40 μL) or 2 (40 μL) was added, and the mixture was incubated in dark at room temperature for 5 minutes to obtain measurement samples with dilution factors of 2 times, 4 times, 8 times, 16 times, and 32 times. The resulting measurement samples had a final concentration of urea of 0.56 M.

(3) Measurement and analysis

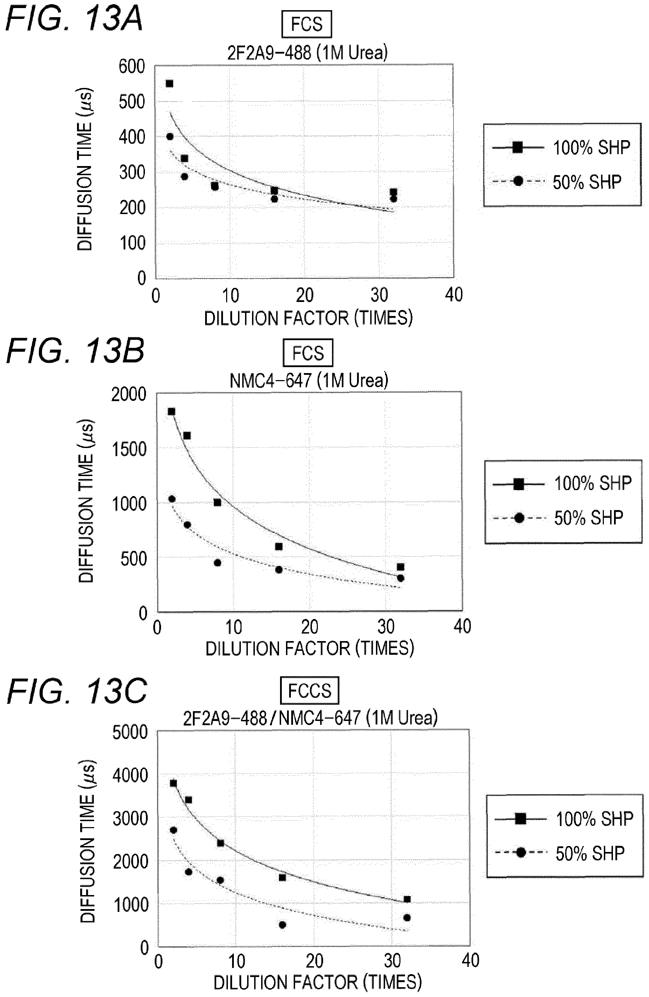

In a similar way as in Example 1, the diffusion time was obtained by FCS or FCCS using an LSM 710 confocal laser microscope (Zeiss) and Zen software (Zeiss). Figs. 13 A to C, 14, and 15 show graphs in which the diffusion times of the respective measurement samples are plotted. Table 7 shows the correspondence between these figures and the reagents, the measurement wavelengths, the fluorescently-labeled antibodies to be detected, and the measurement methods that were used.

[Table 7]

| Figure | Reagent | Measured wavelength (nm) | Fluorescently-labeled antibody/antibodies | Measurement method |
|---|---|---|---|---|
| 13A | Reagent 1 | 488 | 2F2A9-488 | FCS |
| 13B | Reagent 1 | 647 | NMC4-647 | FCS |
| 13C | Reagent 1 | 488/647 | 2F2A9-488/NMC4-647 | FCCS |
| 14 | Reagent 2 | 488 | DakoF-488 | FCS |
| 15 | Reagent 2 | 488/647 | DakoF-488/NMC4-647 | FCCS |

(4) Results

As shown in Figs. 13 A to C, FCS and FCCS using reagent 1 showed that the diffusion times decreased with increasing dilution factors. This indicates that a low VWF concentration in a measurement sample affected the FCS and FCCS measurements. In addition, the higher the dilution factor, the smaller the difference in the diffusion times between the measurement samples derived from 100% SHP and those from 50% SHP. This indicates that the decrease in the VWF concentration in measurement samples reduced the size resolution in the measurements by FCS and FCCS. Therefore, it is suggested that it is unclear whether the information on molecular sizes obtained by FCS and FCCS using fluorescently-labeled monoclonal antibodies as in reagent 1 reflects the accurate molecular size of VWF.

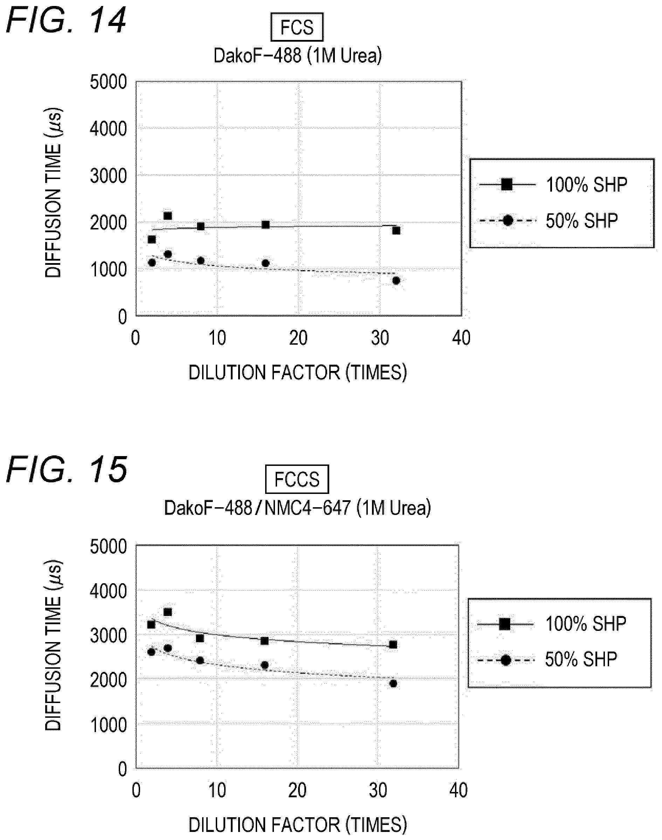

As shown in Fig. 14, FCS using reagent 2 showed that there was little decrease in the diffusion times, even though the dilution factor was increased. As shown in Fig. 15, FCCS using reagent 2 showed that the decrease in the diffusion times due to the increased dilution factors was remarkably reduced. As shown in Figs. 14 and 15, the difference in the diffusion times between the measurement samples derived from 100% SHP and those 50% SHP was maintained at any of the dilution factors. Therefore, it is suggested that the information on molecular sizes obtained by FCS and FCCS using fluorescently-labeled polyclonal antibodies as in reagent 2 reflects the accurate molecular size of VWF.

## Claims

1.　A method for obtaining information on von Willebrand factor (VWF) size, comprising the following steps:

　　denaturing, with urea, VWF contained in a biological sample;
　　fluorescently labeling the denatured VWF using a capturing agent that comprises a fluorescent substance and binds to the denatured VWF; and
　　obtaining information on the size of the fluorescently-labeled VWF by fluorescence correlation spectroscopy or fluorescence cross-correlation spectroscopy;
　　wherein when the information is obtained by fluorescence correlation spectroscopy, the capturing agent comprises a polyclonal antibody, or a plurality of monoclonal antibodies or aptamers that bind to epitopes different from each other; and
　　wherein when the information is obtained by fluorescence cross-correlation spectroscopy, the capturing agent comprises a first capturing agent that comprises a first fluorescent substance and binds to the denatured VWF, and a second capturing agent that comprises a second fluorescent substance and binds to the denatured VWF,

wherein the second fluorescent substance is a fluorescent substance having a maximum fluorescence emission in a wavelength range different from that of the first fluorescent substance, and

wherein the first capturing agent that comprises the first fluorescent substance and binds to the denatured VWF is a polyclonal antibody that comprises the first fluorescent substance, or a plurality of monoclonal antibodies or aptamers that each comprise the first fluorescent substance and bind to epitopes different from each other.

2. The method according to claim 1, wherein obtaining the information comprises obtaining a diffusion time of the fluorescently-labeled VWF by fluorescence correlation spectroscopy or fluorescence cross-correlation spectroscopy.

3. The method according to claim 2, wherein the information is the diffusion time, or a value obtained based on the diffusion time.

4. The method according to claim 1, wherein when the information is obtained by fluorescence cross-correlation spectroscopy, the second capturing agent that comprises the second fluorescent substance and binds to the denatured VWF is a polyclonal antibody that comprises the second fluorescent substance, or a plurality of monoclonal antibodies or aptamers that each comprise the second fluorescent substance and bind to epitopes different from each other.

5. The method according to claim 1, wherein the denaturing is carried out in the presence of urea at a concentration of not less than 0.5 M and not more than 1.75 M.

6. The method according to claim 1, wherein the fluorescent labeling is carried out in the presence of urea at a concentration of not less than 0.2 M and not more than 1 M.

7. A method for preparing a measurement sample for use in fluorescence correlation spectroscopy or fluorescence cross-correlation spectroscopy, comprising the following steps:

denaturing, with urea, von Willebrand factor (VWF) contained in a biological sample; and

fluorescently labeling the denatured VWF using a capturing agent that comprises a fluorescent substance and binds to the denatured VWF;

wherein when the VWF fluorescently labeled with the capturing agent is used for measurement by fluorescence correlation spectroscopy, the capturing agent comprises a polyclonal antibody, or a plurality of monoclonal antibodies or aptamers that bind to epitopes different from each other; and

wherein when the VWF fluorescently labeled with the capturing agent is used for measurement by fluorescence cross-correlation spectroscopy, the capturing agent comprises a first capturing agent that comprises a first fluorescent substance and binds to the denatured VWF, and a second capturing agent that comprises a second fluorescent substance and binds to the denatured VWF,

wherein the second fluorescent substance is a fluorescent substance having a maximum fluorescence emission in a wavelength range different from that of the first fluorescent substance, and

wherein the first capturing agent that comprises the first fluorescent substance and binds to the denatured VWF is a polyclonal antibody that comprises the first fluorescent substance, or a plurality of monoclonal antibodies or aptamers that each comprise the first fluorescent substance and bind to epitopes different from each other.

8. The method according to claim 7, wherein the second capturing agent that comprises the second fluorescent substance and binds to the denatured VWF is a polyclonal antibody that comprises the second fluorescent substance, or a plurality of monoclonal antibodies or aptamers that each comprise the second fluorescent substance and bind to epitopes different from each other.

9. The method according to claim 7, wherein the denaturing is carried out in the presence of urea at a concentration of not less than 0.5 M and not more than 1.75 M.

10. The method according to claim 7, wherein the fluorescent labeling is carried out in the presence of urea at a concentration of not less than 0.2 M and not more than 1 M.

11. Use of a reagent kit in the method according to any one of claims 1 to 10 using fluorescence correlation spectroscopy, the kit comprising urea and a capturing agent that comprises a fluorescent substance and binds to urea-denatured von Willebrand factor (VWF), wherein the capturing agent comprises a polyclonal antibody, or a plurality of monoclonal antibodies or aptamers that bind to epitopes different from each other.

12. The use according to claim 11, wherein the urea is contained in a urea reagent solution, and wherein the concentration of urea in the urea reagent solution is not less than 1 M and mot more than 8 M.

13. Use of a reagent kit in the method according to any one of claims 1 to 10 using fluorescence cross-correlation spectroscopy, the kit comprising urea, a first capturing agent that comprises a first fluorescent substance and binds to urea-denatured VWF, and a second capturing agent that comprises a second fluorescent substance and binds to the urea-denatured VWF, wherein the second fluorescent substance is a fluorescent substance having a maximum absorption in a wavelength region different from that of the first fluorescent substance, and wherein the first capturing agent that comprises the first fluorescent substance and binds to the urea-denatured VWF is a polyclonal antibody that comprises the first fluorescent substance, or a plurality of monoclonal antibodies or aptamers that each comprise the first fluorescent substance and bind to epitopes different from each other.

14. The use according to claim 13, wherein the second capturing agent that comprises the second fluorescent substance and binds to the urea denatured VWF is a polyclonal antibody that comprises the second fluorescent substance, or a plurality of monoclonal antibodies or aptamers that each comprise the second fluorescent substance and bind to epitopes different from each other.

15. The use according to claim 13, wherein the urea is contained in a urea reagent solution, and wherein the concentration of urea in the urea reagent solution is not less than 1 M and mot more than 8 M.

**Patentansprüche**

1. Verfahren zum Erhalten von Informationen über Von-Willebrand-Faktor(VWF)-Größe, umfassend die folgenden Schritte:

   Denaturieren von in einer biologischen Probe enthaltenem VWF mit Harnstoff;
   Fluoreszenzmarkieren des denaturierten VWF unter Verwendung eines Einfangmittels, das eine fluoreszierende Substanz umfasst und an den denaturierten VWF bindet; und
   Erhalten von Informationen über die Größe des fluoreszenzmarkierten VWF durch Fluoreszenz-Korrelationsspektroskopie oder Fluoreszenz-Kreuzkorrelationsspektroskopie;
   wobei das Einfangmittel einen polyklonalen Antikörper oder mehrere monoklonale Antikörper oder Aptamere, die an Epitope binden, die sich voneinander unterscheiden, umfasst, wenn man die Information durch Fluoreszenz-Korrelationsspektroskopie erhält; und
   wobei das Einfangmittel ein erstes Einfangmittel, das eine erste fluoreszierende Substanz umfasst und an den denaturierten VWF bindet, und ein zweites Einfangmittel, das eine zweite fluoreszierende Substanz umfasst und an den denaturierten VWF bindet, umfasst, wenn man die Information durch Fluoreszenz-Kreuzkorrelationsspektroskopie erhält,
   wobei es sich bei der zweiten fluoreszierenden Substanz um eine fluoreszierende Substanz mit einer maximalen Fluoreszenzemission in einem Wellenlängenbereich, der sich von dem der ersten fluoreszierenden Substanz unterscheidet, handelt und
   wobei es sich bei dem ersten Einfangmittel, das die erste fluoreszierende Substanz umfasst und an den denaturierten VWF bindet, um einen polyklonalen Antikörper, der die erste fluoreszierende Substanz umfasst, oder um mehrere monoklonale Antikörper oder Aptamere, die jeweils die erste fluoreszierende Substanz umfassen und an Epitope binden, die sich voneinander unterscheiden, handelt.

2. Verfahren nach Anspruch 1, wobei Erhalten der Information Erhalten einer Diffusionszeit des fluoreszenzmarkierten VWF mittels Fluoreszenz-Korrelationsspektroskopie oder Fluoreszenz-Kreuzkorrelationsspektroskopie umfasst.

3. Verfahren nach Anspruch 2, wobei es sich bei der Information um die Diffusionszeit oder einen Wert, der auf Basis der Diffusionszeit erhalten wurde, handelt.

4. Verfahren nach Anspruch 1, wobei es sich bei dem zweiten Einfangmittel, das die zweite fluoreszierende Substanz umfasst und an den denaturierten VWF bindet, um einen polyklonalen Antikörper, der die zweite fluoreszierende Substanz umfasst, oder um mehrere monoklonale Antikörper oder Aptamere, die jeweils die zweite fluoreszierende Substanz umfassen und an Epitope binden, die sich voneinander unterscheiden, handelt, wenn man die Information durch Fluoreszenz-Kreuzkorrelationsspektroskopie erhält.

5. Verfahren nach Anspruch 1, wobei das Denaturieren in Gegenwart von Harnstoff in einer Konzentration, die nicht weniger als 0,5 M und nicht mehr als 1,75 M beträgt, durchgeführt wird.

6. Verfahren nach Anspruch 1, wobei das Fluoreszenzmarkieren in Gegenwart von Harnstoff in einer Konzentration, die nicht weniger als 0,2 M und nicht mehr als 1 M beträgt, durchgeführt wird.

7. Verfahren zum Herstellen einer Messprobe zur Verwendung bei Fluoreszenz-Korrelationsspektroskopie oder Fluoreszenz-Kreuzkorrelationsspektroskopie, umfassend die folgenden Schritte:

   Denaturieren von in einer biologischen Probe enthaltenem Von-Willebrand-Faktor (VWF) mit Harnstoff; und Fluoreszenzmarkieren des denaturierten VWF unter Verwendung eines Einfangmittels, das eine fluoreszierende Substanz umfasst und an den denaturierten VWF bindet;
   wobei das Einfangmittel einen polyklonalen Antikörper oder mehrere monoklonale Antikörper oder Aptamere, die an Epitope binden, die sich voneinander unterscheiden, umfasst, wenn der mit dem Einfangmittel fluoreszenzmarkierte VWF zur Messung durch Fluoreszenz-Korrelationsspektroskopie verwendet wird; und
   wobei das Einfangmittel ein erstes Einfangmittel, das eine erste fluoreszierende Substanz umfasst und an den denaturierten VWF bindet, und ein zweites Einfangmittel, das eine zweite fluoreszierende Substanz umfasst und an den denaturierten VWF bindet, umfasst, wenn der mit dem Einfangmittel fluoreszenzmarkierte VWF zur Messung durch Fluoreszenz-Kreuzkorrelationsspektroskopie verwendet wird,
   wobei es sich bei der zweiten fluoreszierenden Substanz um eine fluoreszierende Substanz mit einer maximalen Fluoreszenzemission in einem Wellenlängenbereich, der sich von dem der ersten fluoreszierenden Substanz unterscheidet, handelt und
   wobei es sich bei dem ersten Einfangmittel, das die erste fluoreszierende Substanz umfasst und an den denaturierten VWF bindet, um einen polyklonalen Antikörper, der die erste fluoreszierende Substanz umfasst, oder um mehrere monoklonale Antikörper oder Aptamere, die jeweils die erste fluoreszierende Substanz umfassen und an Epitope binden, die sich voneinander unterscheiden, handelt.

8. Verfahren nach Anspruch 7, wobei es sich bei dem zweiten Einfangmittel, das die zweite fluoreszierende Substanz umfasst und an den denaturierten VWF bindet, um einen polyklonalen Antikörper, der die zweite fluoreszierende Substanz umfasst, oder um mehrere monoklonale Antikörper oder Aptamere, die jeweils die zweite fluoreszierende Subtanz umfassen und an Epitope binden, die sich voneinander unterscheiden, handelt.

9. Verfahren nach Anspruch 7, wobei das Denaturieren in Gegenwart von Harnstoff in einer Konzentration, die nicht weniger als 0,5 M und nicht mehr als 1,75 M beträgt, durchgeführt wird.

10. Verfahren nach Anspruch 7, wobei das Fluoreszenzmarkieren in Gegenwart von Harnstoff in einer Konzentration, die nicht weniger als 0,2 M und nicht mehr als 1 M beträgt, durchgeführt wird.

11. Verwendung eines Reagenzkits bei dem Verfahren nach einem der Ansprüche 1 bis 10 unter Verwendung der Fluoreszenz-Korrelationsspektroskopie, wobei das Kit Harnstoff und ein Einfangmittel, das eine fluoreszierende Substanz umfasst und an harnstoff-denaturierten Von-Willebrand-Faktor (VWF) bindet, umfasst, wobei das Einfangmittel einen polyklonalen Antikörper oder mehrere monoklonale Antikörper oder Aptamere, die an Epitope binden, die sich voneinander unterscheiden, umfasst.

12. Verwendung nach Anspruch 11, wobei der Harnstoff in einer Harnstoffreagenzlösung enthalten ist und wobei die Harnstoffkonzentration in der Harnstoffreagenzlösung nicht weniger als 1 M und nicht mehr als 8 M beträgt.

13. Verwendung eines Reagenzkits bei dem Verfahren nach einem der Ansprüche 1 bis 10 unter Verwendung der Fluoreszenz-Kreuzkorrelationsspektroskopie, wobei das Kit Harnstoff, ein erstes Einfangmittel, das eine erste fluoreszierende Substanz umfasst und an harnstoff-denaturierten VWF bindet, und ein zweites Einfangmittel, das eine zweite fluoreszierende Substanz umfasst und an den harnstoff-denaturierten VWF bindet, umfasst, wobei es sich bei der zweiten fluoreszierenden Substanz um eine fluoreszierende Substanz mit einer maximalen Absorption in einem Wellenlängenbereich, der sich von dem der ersten fluoreszierenden Substanz unterscheidet, handelt und wobei es sich bei dem ersten Einfangmittel, das die erste fluoreszierende Substanz umfasst und an den harnstoff-denaturierten VWF bindet, um einen polyklonalen Antikörper, der die erste fluoreszierende Substanz umfasst, oder um mehrere monoklonale Antikörper oder Aptamere, die jeweils die erste fluoreszierende Substanz umfassen und an Epitope binden, die sich voneinander unterscheiden, handelt.

14. Verwendung nach Anspruch 13, wobei es sich bei dem zweiten Einfangmittel, das die zweite fluoreszierende Substanz umfasst und an den harnstoff-denaturierten VWF bindet, um einen polyklonalen Antikörper, der die zweite fluoreszierende Substanz umfasst, oder um mehrere monoklonale Antikörper oder Aptamere, die jeweils die zweite fluoreszierende Substanz umfassen und an Epitope binden, die sich voneinander unterscheiden, handelt.

15. Verwendung nach Anspruch 13, wobei der Harnstoff in einer Harnstoffreagenzlösung enthalten ist und wobei die Harnstoffkonzentration in der Harnstoffreagenzlösung nicht weniger als 1 M und nicht mehr als 8 M beträgt.

**Revendications**

1. Procédé pour obtenir des informations sur la taille du facteur de von Willebrand (VWF), comprenant les étapes suivantes :

   la dénaturation, avec de l'urée, du VWF contenu dans un échantillon biologique ;
   le marquage par fluorescence du VWF dénaturé au moyen d'un agent de capture qui comprend une substance fluorescente et se lie au VWF dénaturé ; et
   l'obtention d'informations sur la taille du VWF marqué par fluorescence par spectroscopie à corrélation de fluorescence ou spectroscopie à corrélation croisée de fluorescence ;
   dans lequel, lorsque l'information est obtenue par spectroscopie à corrélation de fluorescence, l'agent de capture comprend un anticorps polyclonal, ou une pluralité d'anticorps monoclonaux ou d'aptamères qui se lient à des épitopes différents les uns des autres ; et
   dans lequel, lorsque l'information est obtenue par spectroscopie à corrélation croisée de fluorescence, l'agent de capture comprend un premier agent de capture qui comprend une première substance fluorescente et se lie au VWF dénaturé, et un deuxième agent de capture qui comprend une deuxième substance fluorescente et se lie au VWF dénaturé,
   dans lequel la deuxième substance fluorescente est une substance fluorescente ayant une émission de fluorescence maximale dans une plage de longueurs d'onde différente de celle de la première substance fluorescente, et
   dans lequel le premier agent de capture qui comprend la première substance fluorescente et se lie au VWF dénaturé est un anticorps polyclonal qui comprend la première substance fluorescente, ou une pluralité d'anticorps monoclonaux ou d'aptamères qui comprennent chacun la première substance fluorescente et se lient à des épitopes différents les uns des autres.

2. Procédé selon la revendication 1, dans lequel l'obtention de l'information comprend l'obtention d'un temps de diffusion du VWF marqué par fluorescence par spectroscopie à corrélation de fluorescence ou spectroscopie à corrélation croisée de fluorescence.

3. Procédé selon la revendication 2, dans lequel l'information est le temps de diffusion, ou une valeur obtenue sur la base du temps de diffusion.

4. Procédé selon la revendication 1, dans lequel, lorsque l'information est obtenue par spectroscopie à corrélation croisée de fluorescence, le deuxième agent de capture qui comprend la deuxième substance fluorescente et se lie au VWF dénaturé est un anticorps polyclonal qui comprend la deuxième substance fluorescente, ou une pluralité d'anticorps monoclonaux ou d'aptamères qui comprennent chacun la deuxième substance fluorescente et se lient à des épitopes différents les uns des autres.

5. Procédé selon la revendication 1, dans lequel la dénaturation est effectuée en présence d'urée à une concentration non inférieure à 0,5 M et non supérieure à 1,75 M.

6. Procédé selon la revendication 1, dans lequel le marquage fluorescent est effectué en présence d'urée à une concentration non inférieure à 0,2 M et non supérieure à 1 M.

7. Procédé de préparation d'un échantillon de mesure destiné à être utilisé en spectroscopie à corrélation de fluorescence ou spectroscopie à corrélation croisée de fluorescence, comprenant les étapes suivantes :

   la dénaturation, avec de l'urée, du facteur de von Willebrand (VWF) contenu dans un échantillon biologique ; et
   le marquage par fluorescence du VWF dénaturé au moyen d'un agent de capture qui comprend une substance fluorescente et se lie au VWF dénaturé ;

dans lequel, lorsque le VWF marqué par fluorescence avec l'agent de capture est utilisé pour la mesure par spectroscopie à corrélation de fluorescence, l'agent de capture comprend un anticorps polyclonal, ou une pluralité d'anticorps monoclonaux ou d'aptamères qui se lient à des épitopes différents les uns des autres ; et

dans lequel, lorsque le VWF marqué par fluorescence avec l'agent de capture est utilisé pour la mesure par spectroscopie à corrélation croisée de fluorescence, l'agent de capture comprend un premier agent de capture qui comprend une première substance fluorescente et se lie au VWF dénaturé, et un deuxième agent de capture qui comprend une deuxième substance fluorescente et se lie au VWF dénaturé,

dans lequel la deuxième substance fluorescente est une substance fluorescente ayant une émission de fluorescence maximale dans une plage de longueurs d'onde différente de celle de la première substance fluorescente, et

dans lequel le premier agent de capture qui comprend la première substance fluorescente et se lie au VWF dénaturé est un anticorps polyclonal qui comprend la première substance fluorescente, ou une pluralité d'anticorps monoclonaux ou d'aptamères qui comprennent chacun la première substance fluorescente et se lient à des épitopes différents les uns des autres.

8. Procédé selon la revendication 7, dans lequel le deuxième agent de capture qui comprend la deuxième substance fluorescente et se lie au VWF dénaturé est un anticorps polyclonal qui comprend la deuxième substance fluorescente, ou une pluralité d'anticorps monoclonaux ou d'aptamères qui comprennent chacun la deuxième substance fluorescente et se lient à des épitopes différents les uns des autres.

9. Procédé selon la revendication 7, dans lequel la dénaturation est effectuée en présence d'urée à une concentration non inférieure à 0,5 M et non supérieure à 1,75 M.

10. Procédé selon la revendication 7, dans lequel le marquage fluorescent est effectué en présence d'urée à une concentration non inférieure à 0,2 M et non supérieure à 1 M.

11. Utilisation d'un kit de réactifs dans le procédé selon l'une quelconque des revendications 1 à 10 utilisant la spectroscopie à corrélation de fluorescence, le kit comprenant de l'urée et un agent de capture qui comprend une substance fluorescente et se lie au facteur de von Willebrand (VWF) dénaturé par de l'urée, dans laquelle l'agent de capture comprend un anticorps polyclonal, ou une pluralité d'anticorps monoclonaux ou d'aptamères qui se lient à des épitopes différents les uns des autres.

12. Utilisation selon la revendication 11, dans laquelle l'urée est contenue dans une solution de réactif d'urée, et dans laquelle la concentration d'urée dans la solution de réactif d'urée n'est pas inférieure à 1 M et pas supérieure à 8 M.

13. Utilisation d'un kit de réactifs dans le procédé selon l'une quelconque des revendications 1 à 10 utilisant la spectroscopie à corrélation croisée de fluorescence, le kit comprenant de l'urée, un premier agent de capture qui comprend une première substance fluorescente et qui se lie au VWF dénaturé par de l'urée, et un deuxième agent de capture qui comprend une deuxième substance fluorescente et se lie au VWF dénaturé par de l'urée, dans lequel la deuxième substance fluorescente est une substance fluorescente ayant une absorption maximale dans une région de longueur d'onde différente de celle de la première substance fluorescente, et dans lequel le premier agent de capture qui comprend la première substance fluorescente et se lie au VWF dénaturé par de l'urée est un anticorps polyclonal qui comprend la première substance fluorescente, ou une pluralité d'anticorps monoclonaux ou d'aptamères qui comprennent chacun la première substance fluorescente et se lient à des épitopes différents les uns des autres.

14. Utilisation selon la revendication 13, dans laquelle le deuxième agent de capture qui comprend la deuxième substance fluorescente et se lie au VWF dénaturé par de l'urée est un anticorps polyclonal qui comprend la deuxième substance fluorescente, ou une pluralité d'anticorps monoclonaux ou d'aptamères qui comprennent chacun la deuxième substance fluorescente et se lient à des épitopes différents les uns des autres.

15. Utilisation selon la revendication 13, dans laquelle l'urée est contenue dans une solution de réactif d'urée, et dans laquelle la concentration d'urée dans la solution de réactif d'urée n'est pas inférieure à 1 M et pas supérieure à 8 M.

## FIG. 1A

## FIG. 1B

# FIG. 2A

# FIG. 2B

## FIG. 3A

MONOCLONAL ANTIBODY
(2F2A9-488)

## FIG. 3B

MONOCLONAL ANTIBODY
(NMC4-647)

FIG. 4

POLYCLONAL ANTIBODY
(DakoF-488)

FIG. 5

MONOCLONAL
ANTIBODY COCKTAIL
(VWF-488 + SPM-488)

FIG. 6   MONOCLONAL ANTIBODY/
MONOCLONAL ANTIBODY
(2F2A9-488/NMC4-647)

FIG. 7   POLYCLONAL ANTIBODY/
MONOCLONAL ANTIBODY
(DakoF-488/NMC4-647)

## FIG. 8

MONOCLONAL ANTIBODY COCKTAIL/
MONOCLONAL ANTIBODY
(VWF−488+SPM−488/NMC4−647)

## FIG. 9

POLYCLONAL ANTIBODY/
POLYCLONAL ANTIBODY
(DakoF−488/DakoF−647)

# FIG. 10

POLYCLONAL ANTIBODY / POLYCLONAL ANTIBODY
(DakoF-488 / DakoF-647)

# FIG. 11

**FIG. 12A**

FCS
DakoF-488

$y = 18.761x - 96.427$
$R^2 = 0.9661$

**FIG. 12B**

FCS
DakoF-647

$y = 14.66x + 162.7$
$R^2 = 0.9626$

**FIG. 12C**

FCCS
DakoF-488/DakoF-647

$y = 10.002x + 1580.3$
$R^2 = 0.9457$

## FIG. 13A

FCS

2F2A9−488 (1M Urea)

## FIG. 13B

FCS

NMC4−647 (1M Urea)

## FIG. 13C

FCCS

2F2A9−488 / NMC4−647 (1M Urea)

FIG. 14

FCS

DakoF–488 (1M Urea)

FIG. 15

FCCS

DakoF–488 / NMC4–647 (1M Urea)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021173705 A

**Non-patent literature cited in the description**

- **TORRES R. et al.** *Clin. Chem.*, 2012, vol. 58, 1010-1018
- **LIPPOK et al.** *Biophysical Journal*, 2013, vol. 150, 1208-1216
- **GENDRON P-O. et al.** *J. Fluoresc.*, 2008, vol. 18, 1093-1101
- **YAMAMOTO J. et al.** *Opt. Exp.*, 2019, vol. 27, 14835-14841
- **EIGEN M.** ; **RIGLER R.** *Proc. Natl. Acad. Sci. USA.*, 1994, vol. 91, 5740-5747
- **BACIA K. et al.** *Nat. Methods*, 2006, vol. 3, 83-89
- **BACIA K.** ; **SCHWILLE P.** *Nat. Protoc.*, 2007, vol. 2, 2842-2856
- **KRISHEVSKY O.** ; **BONNET G.** *Rep. Prog. Phys.*, 2002, vol. 65 (2), 251
- **BOENDER J. et al.** *Hemasphere*, 2021, vol. 5 (3), e542